# EUROPEAN PATENT SPECIFICATION

(11) **EP 2 683 290 B1**
(45) Date of publication and mention of the grant of the patent: **07.11.2018**
(21) Application number: 12711586.3
(22) Date of filing: 06.03.2012
(51) Int. Cl.: A61B 5/00, G01N 21/64, A61K 49/00

(54) **METHODS FOR IN VIVO TESTING OF THERAPEUTIC ANTIBODIES**
VERFAHREN ZUM IN-VIVO-TESTEN VON THERAPEUTISCHEN ANTIKÖRPERN
PROCÉDÉS DESTINÉS AUX TESTS IN VIVO D'ANTICORPS THÉRAPEUTIQUES

(30) Priority: 07.03.2011 EP 11001857
(43) Date of publication of application: 15.01.2014
(73) Proprietor: F. Hoffmann-La Roche AG, 4070 Basel (CH)
(72) Inventor: DOBOSZ, Michael, 82393 Iffeldorf (DE); SCHEUER, Werner, 82377 Penzberg (DE)
(74) Representative: Schiweck Weinzierl Koch Patentanwälte Partnerschaft mbB
(86) International application number: PCT/EP2012/053786
(87) International publication number: WO 2012/119999

(56) References cited:
- H. J.W.L. AERTS ET AL: "Disparity Between In Vivo EGFR Expression and 89Zr-Labeled Cetuximab Uptake Assessed with PET", THE JOURNAL OF NUCLEAR MEDICINE, vol. 50, no. 1, 17 December 2008 (2008-12-17), pages 123-131, XP55026510, ISSN: 0161-5505, DOI: 10.2967/jnumed.108.054312
- NICHOLE R. DEAN ET AL: "Use of Optical Imaging to Predict Tumor Response to Anti-EGFR Therapy", THE LARYNGOSCOPE, vol. 119, no. S1, 1 January 2009 (2009-01-01), pages S63-S63, XP055004328, ISSN: 0023-852X, DOI: 10.1002/lary.20334
- EMILY HELMAN ET AL: "Optical imaging predicts tumor response to anti-EGFR therapy.", CANCER BIOLOGY & THERAPY, vol. 10, no. 2, 1 July 2010 (2010-07-01), pages 166-171, XP055004310, ISSN: 1538-4047, DOI: 10.4161/cbt.10.2.12164
- JOHN GLEYSTEEN ET AL: "Fluorescently labeled cetuximab to evaluate head and neck cancer response to treatment.", CANCER BIOLOGY & THERAPY, vol. 6, no. 8, 1 August 2007 (2007-08-01), pages 1181-1185, XP055004331, ISSN: 1538-4047
- MACOR P ET AL: "Development of a human-SCID lymphoma as a model to evaluate the therapeutic effect of Rituximab", MOLECULAR IMMUNOLOGY, PERGAMON, GB, vol. 45, no. 16, 1 October 2008 (2008-10-01), page 4163, XP025896375, ISSN: 0161-5890, DOI: 10.1016/J.MOLIMM.2008.08.204 [retrieved on 2008-10-01]

## Description

The present invention provides methods and means for identifying a cancer-patient disposed to respond favourably to a therapeutic antibody as well as methods for in vivo selecting a xenograft tumor model which is most qualified for preclinical testing of a therapeutic antibody. These methods apply near-infrared fluorescence imaging which is an excellent imaging technology because of its simplicity, fast scanning times and non-hazardous radiation.

An animal model is a living, non-human animal used during the research and investigation of human disease, for the purpose of better understanding the disease without the risk of causing harm to an actual human being during the process. The animal chosen will usually meet a determined taxonomic equivalency to humans, so as to react to disease or its treatment in a way that resembles human physiology as needed. Many drugs, treatments and cures for human diseases have been developed with the use of animal models.

Mouse models of cancer have since long been used to qualify new anti-cancer drugs for the development of human clinical trials. The most used models are xenografts of human tumors grown subcutaneously in immunodeficient mice such as athymic (nude) or severe combined immune deficient (SCID) mice (Sausville and Burger, Cancer Res. (2006), 66: 3351-3354; Kerbel, Cancer Biology & Therapy 2:4: Suppl.1 (2003): S134-S139; Troiani et al., Crit. Rev. Oncol/Hematol (2008), 65: 200-211). Specifically Helman et al. (2010) "Optical imaging predicts tumor response to anti-EGFR therapy" Cancer Biology & Therapy, Vol. 10, Issue 2, p. 166-171 inter alia evaluated cetuximab treatment in head and neck squamous cell carcinoma xenografts.

The testing of a new potential medicament requires preclinical testing of the medicament in animals. Specifically, once a molecule was elected to be tested as a medicament it is important to have available the best qualified animal model. In case of testing an anti-tumor medicament for humans, it is required to have available the most appropriate xenograft tumor model. In fact, the relevance of each particular model depends on how close it replicates the histology, physiological effects, biochemical pathways and metastatic pattern observed in the same human tumor type. The generation and selection of the most appropriate xenograft model is thus of utmost importance. Specifically in case of testing anti-cancer antibodies, it is highly desirable to have available the most appropriate xenograft model.

Scientists thus developed many models for the selection of compounds and treatments that go into clinical testing of patients, including syngeneic models, human tumor xenograft models, orthotopic models, metastatic models, transgenic models, and gene knockout models. However, while much effort was put into the development of models, no efficient strategy is available to adapt the model to the needs required for testing the medicament, for example, an antibody. In particular, though many models are available, no criteria are available that would allow scientists to elect the model (xenograft model) that is most appropriate for their testing of, for example, a potential therapeutically effective antibody. Thus it may happen that the wrong (i.e. incompatible) xenograft model is chosen, though better ones may have been available. However, the wrong xenograft model may have been chosen, because it was known that it expresses the target of the antibody in high amounts and it may have shown good performance in *in vitro* analysis of antibody-target interactions as determined by immunohistochemistry. Accordingly, though a more appropriate model may have been available, it would not have been chosen because means and methods were not at hand which allowed the selection of the most appropriate xenograft model.

Hence, the technical problem of the present invention is to comply with the needs described above.

The present invention addresses these needs and thus provides as a solution to the technical problem the embodiments concerning means, for example, tools and kits as well as methods and uses applying these means for in vivo selecting a xenograft tumor model which is most qualified for preclinical testing of a therapeutically effective antibody. Similarly, the present invention provides means and methods for identifying a cancer-patient disposed to respond favorably to a therapeutic antibody.

These embodiments are characterized and described herein, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

As described herein, "preferred embodiment" means "preferred embodiment of the present invention". Likewise, as described herein, "various embodiments" and "another embodiment" means "various embodiments of the present invention" and "another embodiment of the present invention", respectively.

The inventors of the present application with the aim of selecting a xenograft tumor model which is most qualified for preclinical testing of a therapeutically effective antibody developed the methods of the present invention described herein which allow the selection of the most qualified xenograft tumor model.

Accordingly, the present invention provides a non-invasive method of in vivo selecting a xenograft tumor model which is most qualified for preclinical testing of a therapeutically effective antibody comprising
(a) detecting in one or more xenograft tumor models said therapeutically effective antibody which is fluorescence labeled after binding to its target in said xenograft tumor model by near-infrared fluorescence imaging (NIRF); and
(b) selecting the xenograft tumor model which shows after binding of said therapeutically effective antibody to said target in said xenograft tumor model the highest fluorescence signals.

Accordingly and in essence, the method of the present invention is for in vivo selecting a xenograft tumor model which is most qualified (i.e., appropriate) for preclinical testing of a therapeutically effective antibody. It is likewise for optimizing a xenograft tumor model for preclinical testing of a therapeutically effective antibody.

Also, it can be applied for personalizing an antibody-based therapy in that a tumor, preferably including histologically-intact fragments (including cells, tumor tissue) of a human cancer, is taken directly (explanted) from a human patient and is implanted into a non-human mammal (preferably a mouse or rat) to the corresponding location (including the respective organ of the non-human animal). Accordingly, a therapeutically effective antibody can be tested as to whether or not it would be most effective for an antibody-based therapy. Thus, such an orthotopic model is deemed to reflect the in vivo situation of a tumor in a human; see elsewhere herein for further details. However, the methods of the present invention are not limited to a xenograft tumor model, but are also applicable to identifying a cancer-patient which is disposed to respond favorably to a therapeutic antibody. Put it differently, the method of the present invention also serves for the purpose of patient stratification as described in detail herein below. In addition, labeled antibodies can be applied for intra-operative surgery. This near-infrared guided surgery supports the surgeon in identifying tumor tissue. Furthermore, labeled antibodies can support diagnosis of tumor tissue based on endoscopic procedures.

Thus, the present invention enables the tailoring of an antibody-based therapy in that one or more xenograft tumor models are generated such as one in which the tumor grows subcutaneously or an orthotopic xenograft tumor model and the one which is then most qualified is selected for testing the therapeutically most effective xenograft tumor model by performing the methods of the present invention.

Further, the methods of the present invention allow the testing as to whether a panel of potential xenograft tumor models comprising at least two, three, four, five, six, seven, eight, nine, ten, fifteen, twenty, thirty, forty, fifty, or more xenograft tumor models is suitable for preclinical testing of a therapeutically effective antibody. Specifically, the methods described herein allow the simultaneous testing of two, three, four, five, six, seven, eight, nine, ten, fifteen, twenty, thirty, forty, fifty, or more xenograft tumor models.

The methods and uses described herein allow real-time in vivo selection of a xenograft tumor model which is most qualified for preclinical testing of a therapeutically effective antibody, i.e., no in vitro step (such as immunohistochemistry or the like) in selecting is required and/or no delay in generating and collecting data such as developing pictures or the like will occur. Accordingly, any change in the performance of the therapeutically effective antibody may be directly (online) visualized.

An advantage of the method of the present invention is thus that it allows imaging under real time conditions the performance of the xenograft tumor model for the preclinical testing of potentially therapeutic antibodies in vivo so as to allow the selection of the most qualified xenograft tumor model. Accordingly, the method of the present invention allows the in vivo simulation of the performance of a therapeutically effective antibody on its target, since the most qualified xenograft tumor model is deemed to reflect the in vivo conditions present in a human.

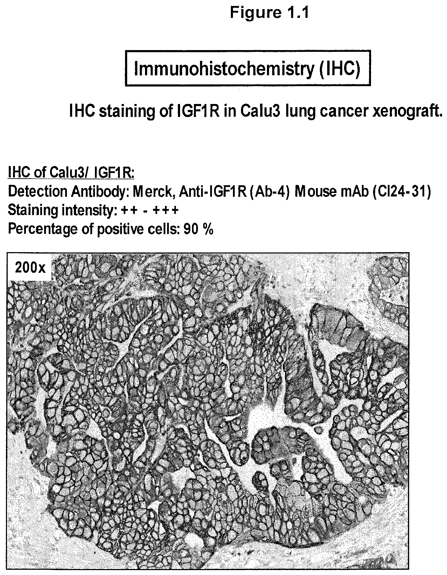

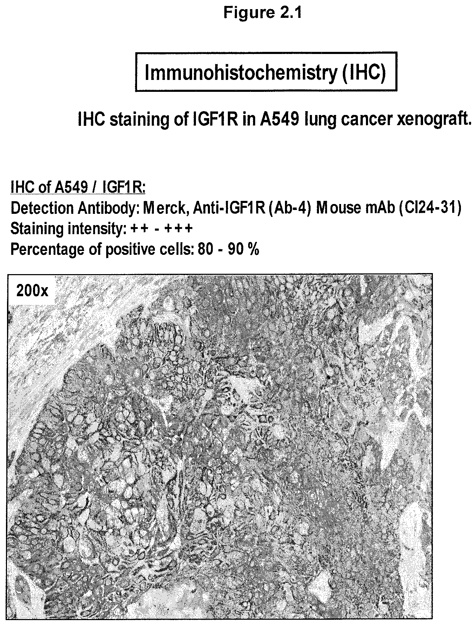

In fact, the inventors found that xenograft tumor models, which were deemed to be most qualified for preclinical testing of a therapeutically antibody because these models showed good results in in vitro tests such as IHC and/or were known to express the target of the therapeutically effective antibody, did not show preclinical activity in the model in that it did, for example, not properly bind to its target and/or did not sufficiently activate immune cells; see Figures 1.1 to 1.3 and 2.1 to 2.3.

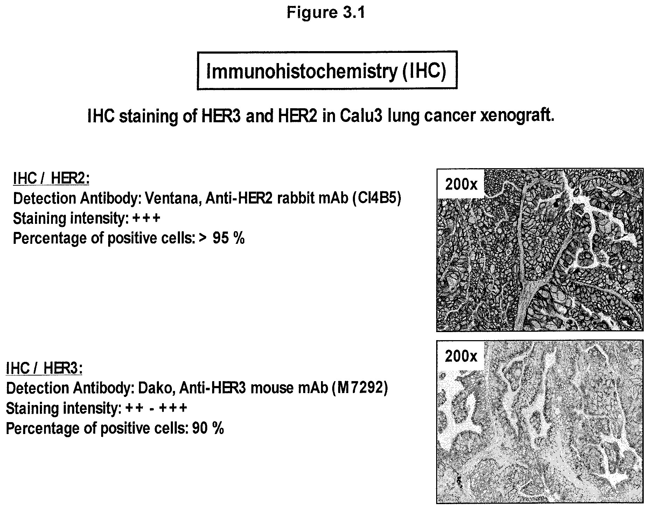

Likewise, the inventors found that xenograft tumor models, which were deemed to be not qualified for preclinical testing of a therapeutically antibody because these models showed disappointing or even unsatisfying results in in vitro tests such as IHC, were suitable for preclinical testing of a therapeutically effective antibody in the model; see Figures 3.1 to 3.4

In particular, the inventors rather than applying standard in vitro tests for selecting and/or verifying the suitability of a xenograft tumor model for preclinical testing of a therapeutically effective antibody applied near-infrared fluorescence (NIRF) imaging to monitor the performance of the xenograft tumor model and made the afore described findings which are illustrated in the appended Examples.

Hence, the inventors concluded that the commonly applied in vitro techniques for selecting the most qualified xenograft tumor model for preclinical testing of a therapeutically effective antibody are not reliable, while in vivo monitoring for selecting the most qualified xenograft tumor model is more reliable. Accordingly, the inventors developed the methods of the present invention.

The method of the present invention is also applicable to select a xenograft tumor model for (preclinical) testing the diagnostically most effective antibody. Accordingly, as far as a diagnostic aspect is concerned, in all embodiments described herein, the term "therapeutically" or "therapeutic" or any other grammatical form thereof is to be understood to mean "diagnostically" or "diagnostic" or any grammatical form thereof. By way of example, a therapeutically effective antibody is then a diagnostically effective antibody.

It is known that various xenograft tumor models of tumors (or cancers) are available; see the mouse models of human cancers consortium (MMHCC) at the National Cancer Institute (NCI) (http://emice.nci.nih.gov/mouse_models/). For example, various mouse models such as gastrointestinal cancer models, hematopoietic cancer models, lung cancer models, mammary gland cancer models, nervous system cancer models, ovarian cancer models, prostate cancer models or skin cancer and melanoma models are available which can be used for preclinical testing of drugs such as antibodies.

However, not every xenograft tumor model is suitable for testing a drug, in particular an antibody. This is so because, the testing of an antibody includes whether or not it may bind its target. The binding of an antibody to its target may, however, depend on various factors such as sufficient expression of its target so that a sufficient number of target molecules is provided by a cell of the xenograft tumor to the antibody, the condition of the target such as its glycosylation status, whether or not it may be shedded or buried, etc. as described herein elsewhere.

Accordingly, it is decisive to select the most qualified xenograft tumor model for the preclinical testing of a therapeutically effective antibody. The present invention therefore provides methods and means for in vivo selection of the most qualified xenograft tumor model for the preclinical testing of a therapeutically effective antibody by applying NIRF methodology as described herein. As mentioned herein, many xenograft tumor models are available for preclinical testing a therapeutically effective antibody. Accordingly, the most qualified xenograft tumor model that is selected in accordance with the methods and means of the present invention is preferably selected out of a panel of potential xenograft tumor models.

A "panel of potential xenograft tumor models may include at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 85, 90, 95, 100 or more xenograft tumor models.

The methods of the present invention are non-invasive. "Non-invasive" as used herein means that the methods, uses and/or kits/devices of the invention do not create skin and/or mucosa breaks of said subject, but do allow and involve contact of the skin/mucosa, with radiation and, likewise, penetration of the skin/mucosa and all subsequent layers by radiation. Radiation thereby includes all kinds of light such as e.g. excitation light, emission light etc. which is described herein in the context of the present invention.

The term "in vivo" includes selecting the most qualified xenograft tumor model for preclinical testing of a therapeutically effective antibody under real-time conditions. It thus refers to localizing and/or detecting a potentially therapeutic effective antibody using an imaging or scanning technology in a living non-human mammal (test animal).

Accordingly, the performance of an antibody in a xenograft tumor model can be monitored in a living non-human mammal (test animal). Hence, the methods of the present invention are not carried out in a partial or dead organism. The in vivo selection of a xenograft tumor model is thus deemed to reflect the scenario that occurs/is present in a human, thereby it takes into account the naturally-occurring conditions in a subject.

Therefore, the methods of the present invention allow selecting the most qualified xenograft tumor model that is most appropriate for preclinical testing of therapeutically active antibodies. For example, the target antigen of the therapeutically effective antibody may be differently expressed in terms of its expression level in a xenograft tumor model, may be buried, unusually glycosylated, etc., thereby creating differences among xenograft tumor models. However, the methods of the present invention overcome these potential problems, thereby making available the selection of the most qualified xenograft tumor model for the preclinical testing of a therapeutically effective antibody which is preferably intended to be applied in an antibody-based therapy.

In the present invention, an antibody that is tested for its preclinical activity in the most qualified xenograft tumor model selected by the methods of the present invention is fluorescence labeled.

Preferably, the therapeutically active antibody may be selected from a panel of antibodies that may be therapeutically active. A "panel" of potentially therapeutic effective antibodies may include at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 85, 90, 95, 100, 120, 140, 150, 160, 180, 200, 220, 240, 250, 260, 280, 300, 400, 500, 600, 700, 800, 900 or 1000 antibodies. Accordingly, the antibodies comprised by the panel of potentially therapeutic effective antibodies are fluorescence labeled, i.e., each of the antibodies comprised by said panel is fluorescence labeled.

Without being bound by theory, it is envisaged that the antibodies comprised by the panel, though binding the same desired target, are different from each other. For example, they may bind to different epitopes of said target or may bind with different affinity to said target.

In a preferred embodiment, the panel of potentially therapeutic effective antibodies comprises antibodies intended to be used in therapy of a subject, preferably a human.

A subject when used herein includes mammalian and non-mammalian subjects, with mammalian subjects being preferred and human being particularly preferred. "Mammal" for purposes of the invention refers to any animal classified as a mammal, including human, domestic and farm animals, non-human primates, and any other animal that has mammary tissue. A mammal includes human, rodents such as mouse, rat, guinea pig, rabbit, dog, cat, horse, camel, pig, cow, goat, chimpanzee etc., with human being preferred. A subject also includes human and veterinary patients.

The term "xenograft tumor model" when used in the context of the present invention is also referred to herein as "xenograft cancer model" or "xenograft model". Thus, these terms can be equivalently used.

A "xenograft" when used herein is a tissue or organ from a subject of one species transplanted into or grafted onto an organism of another species, genus, or family. In the present invention a xenograft is preferably from a human subject transplanted into or grafted onto a non-human subject, preferably a non-human mammal, preferably a rodent such as a rat, mouse, guinea pig, hamster, with mouse being preferred, or a non-human primate such as a chimpanzee or a macaque.

The tissue or organ is preferably from a tumor comprising malignant/cancerous cells as described herein. The tumor is preferably from a human. Preferably, the tumor includes histologically-intact fragments. Fragments include tumor cells and/or tumor tissue.

The xenograft tumor model of the methods of the present invention is a non-human mammal (sometimes also referred herein as "test animal"). The non-human mammal is preferably a rodent. More preferably, the rodent is mouse, rat, guinea pig, or hamster. The non-human mammal can also be a rabbit, dog, cat, horse, camel, pig, cow, goat, or a non-human monkey such as a chimpanzee or macaque.

The test animal is a recipient non-human mammal (sometimes also referred to herein as "recipient non-human mammal" or "recipient xenograft tumor model") onto which is grafted or into which is transplanted a xenograft which, in the context of the present invention, is a tumor. The recipient non-human mammal is capable of receiving the xenograft. Accordingly, it is preferably immuno-compromised and/or is preferably mostly incapable of mounting a graft-rejection immune response thereby accepting the foreign tissue as self.
Examples of immuno-compromised animals include: severe combined immuno deficient (SCID) mice, SCID/beige mice, nude mice, NIH-nu-bg-xid mice, etc.

The xenograft tumor comprised by test animal preferably replicates the tumor histology, physiological effects, biochemical pathways and metastatic pattern observed in the same human tumor type. Accordingly, the xenograft tumor model allows the study of tumor growth, metastasis, etc.

The term "immuno-compromised" is used to describe a recipient non-human mammal in which the immune system has been partly or completely suppressed in order to allow engrafted foreign cells or tissue to grow with minimal chance of rejection by the recipient non-human mammal. Particularly, the term immuno-compromised is used to describe a non-human mammal that is partially or completely immuno-suppressed by biological means or chemical means. Chemical immuno-suppressive agents are well known in the art, especially repeated treatment with cyclosporin. The term immuno-compromised also includes non-human mammals that are partially or completely immuno-deficient. These non-human mammals include rodents having no functional T-cell, and/or B cell and/or NK cell immunity. Further non-limiting examples of such rodents are: nude mice, severe combined immuno deficient (SCID) mice, SCID/beige mice, or NIH-nu-bg-xid mice. Still other immune compromised or deficient mice, rodents, or other animals may be used, including those which are deficient as a result of a genetic defect, which may be naturally occurring or induced, such as, for example, Balb/c nude mice, CD-I, Fox Chase SCID, MRL, NIH-III, NOD-SCID, Nu/Nu, Swiss nude, and the like, and mice which have been cross-bred with these mice and have an immune-compromised background. In addition to mice, immune deficient rats or similar rodents may also be employed in the practice of the invention.

A human xenograft tumor model is thus generated by grafting onto or transplanting into a histologically-intact fragment of a tumor, preferably a human tumor (including cells, tissue, etc.) into an immune deficient non-human mammal (test animal), preferably a rodent such as a mouse, rat, hamster, guinea pig, with mouse being preferred. Also, primary tumor cells or primary tumor cell line, preferably from a human is grafted into an immune deficient non-human mammal. Further, also an established tumor cell line, preferably from a human, can be grafted into an immune deficient non-human mammal.

"Grafting" refers to inoculating, implanting, transplanting or transferring tissue or cells in a non-human mammal from one area of a subject (donor), preferably human subject to the non-human mammal (recipient).

For example, implantation can be achieved by injection. Such xenografts may be introduced alone or in conjunction with a basement membrane composition, such as Matrigel (BD Biosciences), an extracellular matrix preparation, which has been shown to enhance the growth of tumor cells in vitro.
The grafting may be cutaneous grafting. "Cutaneous grafting" refers to implanting tissue on the surface of the skin.
The grafting may be sub-cutaneous grafting. "Sub-cutaneous grafting" refers to the implanting of tumor cells and/or tumor tissue under the skin of a recipient xenograft model. The graft site is preferably completely enclosed with the recipient's or host's skin tissue. Accordingly, a xenograft is implanted via subcutaneous injection into the flank of the recipient non-human mammal, for example, a nude mice.
Grafting also includes orthotopic grafting. In "orthotopic grafting" tumor tissue and/or cells are implanted at the site of the organ of origin. This organ-specific site presumably provides the tumor cells with an optimal environment for growth and progression and may reflect the clinical situation most closely.

Various subcutaneous xenograft tumor models are available (the cell line used in these models in indicated in parentheses): breast (MCF-7, MDA-MB-231, SK-BR-3, BT474, MDA-MB-231,MDA-MB-435, MDA-MB-453, MDA-B-468, MDA-MB-469, MX-1, T-47D, ZR-75-1), prostate (DU-145, PC-3, LNCaP cells), Colorectal (HT-29, HCT-116, DLD-1, SW620, LoVo, Colo-205, SW480, Colo-320DM, HCT 116, HCT 8 cells), Lung (A549, NCI-H460, SK-MES-1, NCI-H226, Calu-3, Calu-6, H23, h249, H345, H460, H522, H125, H1299, H1703, H1975, N-592, SK-MES-1, WI-38 cells), Glioblastoma/Brain (9L, C6, D54, DBTRG, SF-295, SF-539, SF767, SNB-19, U87MG, U251, U-373MG cells), Fibrosarcoma (HT-1080 cells), Pancreatic (Mia-Paca-2, Bx-PC-3, Capan-1, Capan-2, Capan-3, pa/III, Pan 02, Pan 03, Panc-1, Su.86.86, AsPc-1 cells), Kidney (786-O, A498 cells), Liver (HepG2, SK-HEP-1, Hep3B, PLC/PRF/5 cells), Osteosarcoma (143B, SJSA-1, Saos-2 cells), Melanoma (A375, SK-MEL-5, A2058 cells), Nasopharyngeal (CNE2 cells), Gastric (MGC803, BGC823, NCI-N87 cells), Ovarian (C-33 A, SK-OV-3, OVCAR-3, OVCAR-4, OVCAR-5 cells), Leukemia (K562, HL-60, MV-4-11 cells), Lymphoma (Namalwa, Daudi cells), Multiple myeloma (RPMI-8226 cells), ALL (MOLT-4), AML/CML (HL60, KG1, OPM-2, P388, THP-1), Oral cancers (KB cells), Bladder (T24, 5637 cells), Head7Neck (HONE-T-1), Skin (A375, A431, b16, Lox IMVI, Malme-3m, WM-115), Stomach (N87), Uterine (DX5).

Various orthotopic xenograft tumor models are available: Breast (BT474, MDA-MB-231 MCF-7, MCF-7(her2+) cells), Prostate (LNCaP, DU-145 cells), Lung (A549, H1975 cells), Glioblastoma (U87MG, LN-229 cells), Pancreatic (MiaPaCa-2, PANC-1 cells), Kidney (786-O cells), Liver (HepG2 cells).

The aforementioned xenograft tumor models are non-exhaustive examples of the panel of xenograft tumor models as described herein elsewhere.

Although useful, without being bound by theory, xenograft tumor models of human cancer could be improved to even more closely replicate the real disease. For example, xenografts may show loss of the normal tumor architecture and often consist of a dominant clone that was not evident in the primary tumor. Moreover, the vascular and lymphatic systems may not be well established in xenografts and there may be aberrant immune response.

Accordingly, a xenograft tumor model may be engineered by genetic engineering with the aim of achieving a humanization of the xenograft tumor model. For example, a transgene can be overexpressed such as an oncogene or dominant negative tumour-suppressor gene within a specific tissue through the use of ectopic promoter and enhancer elements, such as the immunoglobulin heavy chain enhancer in Eµ-Bcl2 or Eµ-Myc transgenics. The capacity to regulate the function of a transgene through the use of exogenous ligands, such as doxycycline to regulate transcription (the Tet system), or tamoxifen to regulate protein function, have enabled the temporal regulation of oncogene expression and the demonstration of 'oncogene addiction' in a tissue. For example, the regulation of K-ras and H-ras by doxycycline demonstrated a role for these oncoproteins in the induction and maintenance of lung cancer and melanoma, respectively.

Accordingly, it is envisaged to genetically engineer xenograft tumor cells by introducing mutations in oncogenes such as K-RAS, H-RAS, N-RAS, EGFR, MDM2, RhoC, AKT1, AKT2, c-myc, n-myc, ss-catenin, PDGF, C-MET, PI3K-CA, CDK4, cyclin B1, cyclin D1, estrogen receptor gene, progesterone receptor gene, Her2, ErbB1, ErbB3, ErbB4), TGFxx, TGF-ss, ras-GAP, Shc, Nck, Src, Yes, Fyn, Wnt, Bel2 PyV MT, and/or SV40 LT.

It is alternatively and or additionally envisaged to genetically engineer xenograft tumor cells by introducing mutations in tumor-suppressor genes such as Rb, P53, INK4a, PTEN, LATS, Apafl, Caspase 8, APC, DPC4, KLF6, GSTP1, ELAC2/HPC2 or NKX3.1, ATM, CHK2, ATR, BRCA1, BRCA2, MSH2, MSH6, PMS2, Ku70, Ku80, DNA/PK, XRCC4 or MLH1), NF1, NF2, APC, WT, Patched, and FHIT.

Such technologies also enable the loss of tumor-suppressor genes in cancer, such as those observed in human familial syndromes, to be replicated in a non-human mammal, preferably a rodent, more preferably mice. Conditional models are also envisaged. These enable the deletion or expression of a gene within a specific tissue, under the control of their endogenous promoter through the use of recombinases such as Cre-Lox and FLP-FRT. This can also be combined with ligand-dependent activation of Cre through the use of the tamoxifen-dependent Cre-ERT to achieve greater temporal control. Any of the aforementioned oncogenes may be conditionally (over)expressed and/or any of the aforementioned tumor-suppressor genes may be conditionally deleted.

Accordingly, a non-human mammal, preferably a rodent, more preferably mice is/are being engineered that harbor human genomic loci including non-coding regulatory elements, genes involved in the immune response and genes that regulate drug metabolism and protein glycosylation. All of these modifications should enable mRNA profiles, cellular and serum biomarkers, changes in tumor metabolism and relevant alterations in the tumor microenvironment to more closely model changes that occur during tumorgenesis in humans, and therefore should translate much more effectively into the clinic.

Accordingly, it is envisaged that the xenograft tumor models referred to herein are preferably genetically modified in accordance with the aforementioned methodology to become more humanized xenograft tumor models. Likewise, the xenograft tumor models can be established in that primary human tumor cells are grafted or implanted, for example, as fresh human tumor fragments into an immune-deficient non-human mammal, preferably a rodent, more preferably into mice. Compared with cell line based traditional xenograft tumor models, without being bound by theory, primary models may better mimic original patient histopathology, as well as gene expression profile, and may therefore better provide predictive value for cancer indication prioritization and clinical efficacy estimation. The following are non-exhaustive examples of primary human tumor models, including gastric, colorectal, liver, lung, head and neck, and kidney cancer models available from Crown Bioscience Inc. (www.crownbio.com). Of course, xenograft tumor models can also be established by means and methods known in the art and can be based on any primary tumor cells obtained from any tumor including those mentioned herein.

However, if the therapeutically effective antibody may act through the immune system, a tumor model established in, for example, nude or SCID mice may be impractical. Indeed, the lack of T cell function in nude and SCID mouse xenograft models makes it impractical for testing such therapeutics. Accordingly, a rodent, preferably a murine syngeneic tumor model is then most appropriate to facilitate research.

Accordingly, it is also envisaged that the methods of the present invention are applied for the in vivo selection of the most qualified syngeneic tumor model which is most qualified for preclinical testing of a therapeutically effective antibody. The method steps and embodiments described herein are equally applicable to this aspect.
A syngeneic tumor model is, for example, created by inoculating a mouse or rat tumor cell line into the corresponding immune competent mouse or rat strain.
Syngeneic tumor models for lung (LLC cells), liver (H22 cells), Kidney (Renca cells) or colon (CT-26 cells) are available and are envisaged to be applied in the methods of the present invention.

Generally, both xenograft and syngeneic tumor models provide a consistent and reproducible tool for evaluating tumor cell growth, as well as permit easy access to the mass for tumor treatment and ultrasound evaluation. Metastatic tumor incidence can be monitored through radiography, high-resolution ultrasound imaging, and necropsy.

In the embodiments of the invention the non-human mammal comprises a xenograft, preferably a human xenograft, said xenograft being a (human) tumor (xenograft (human) tumor). Accordingly, the xenograft tumor model applied in the methods and uses of the present invention comprises a tumor. The tumor is from a species different from the xenograft model species. Preferably, the xenograft model comprises a human tumor.

Accordingly, in preferred embodiments the tumor is from a human. The term "tumor" includes solid tumors, cancer cell lines established either from a subject, preferably established from human subject or already established, known cell lines, tissue, etc. Solid tumors may be benign (not cancer), or malignant (cancer).

The term "solid tumor" when used herein refers to tumors elected from the group of gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer, preferably breast cancer.

For the purpose of the present invention, a solid tumor has a size of at least 1 mm, preferably 2 mm. At this size, a solid tumor in order to proliferate and to survive depends on an adequate supply of growth factors and the removal of toxic molecules as well as on a sufficient oxygen supply. In solid tissues, oxygen, for example, can diffuse radially from capillaries for only about 150 to 200 µm and, thus, the tumor depends on the supply of oxygen by blood vessels and, thus, initiates angiogenesis.

For example, a solid tumor refers to a tumor selected from the group of gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer.

When used herein the term "cancer" refers to a medical condition that is characterized by the growth of a tumor. It is thus the physiological condition in a subject that is typically characterized by unregulated cell growth.

The tumor comprises preferably malignant/cancerous cells having a (desired) target for the therapeutically active antibody on their surface.

The (desired) target on the surface of a mammalian cell is preferably a molecule or a complex of molecules.

The "surface" of a cell includes the cell membrane (also called the plasma membrane). It is the biological membrane separating the interior of a cell (preferably a mammalian cell) from the outside environment.
The cell membrane surrounds all cells and it is semi-permeable, controlling the movement of substances in and out of cells. It contains a wide variety of biological molecules, primarily lipids and proteins. These proteins include transmembrane proteins, lipid anchored proteins and peripheral proteins which are preferred targets in the sense of the present invention.

"Transmembrane proteins" span the membrane and have a hydrophilic cytosolic domain, which interacts with internal molecules, a hydrophobic membrane-spanning domain that anchors it within the cell membrane, and a hydrophilic extracellular domain that interacts with external molecules.
"Lipid anchored proteins" are covalently-bound to single or multiple lipid molecules; hydrophobically insert into the cell membrane and anchor the protein.
"Peripheral proteins" are attached to integral membrane proteins, or associated with peripheral regions of the lipid bilayer.

In accordance with the teaching of the present invention, proteins which extend to the exterior of a cell such as receptor-molecules are typically available as an antigen that can be targeted by antibodies. These proteins are preferred. Accordingly, the target is preferably a protein having an extracellular portion.

The term "receptor-molecule" relates thus to a protein on the cell membrane or within the cytoplasm or cell nucleus that binds to a ligand and typically transduces a signal, such as metabotropic receptors, G protein-coupled receptors, guanylyl cyclise receptors, receptor tyrosine kinases, muscarinic acetylcholine receptors, adenosine receptors, adrenoceptors, GABA receptors, angiotensin receptors, cannabinoid receptors, cholecystokinin receptors, dopamine receptors, glucagon receptors, metabotropic glutamate receptors, histamine receptors, olfactory receptors, opioid receptors, chemokine receptors including cytokine receptors, calcium-sensing receptor, somatostatin receptors, serotonin receptors, secretin receptors, ionotropic receptors, or Fc receptors.

Further preferred targets are CA 15-3, CA 19-9, CA 72-4, CFA, MUC-1, MAGE, p53, ETA, CA-125, CEA, AFP, PSA, PSMA, or OE8.

A tumor cell or tumor cell line detected in the present invention is a mammalian cell characterized by unregulated cell growth. It may include all types of cells, for example, cells of organs, muscle cells, nerve cell, cells of the immune system (lymphocytes including PBMCs, B-cells, T-cells, NK cells, macrophages), eosinophils, neutrophils, basophils. In preferred embodiments, said mammalian cell is a malignant cell.

"Malignant" describes a cell that contributes to a progressively worsening disease, in particular growth of a tumor or the diseases described herein. The term is most familiar as a description of cancer including growth of tumor. Malignant cells are not self-limited in their growth, are capable of invading into adjacent tissues, and may be capable of spreading to distant tissues (metastasizing). Malignant when used herein is synonymous with cancerous.

"Preclinical testing" includes the in vivo testing of a therapeutically effective antibody in a non-human (test) mammal (test animal) to gather information for a clinical trial. This information includes the collection and evaluation of pharmacology, toxicology, pre-formulation, formulation, pharmacokinetics, pharmacodynamics data. Accordingly, the test animal will deliver data on typical parameters of antibodies such as the efficacy of its binding to the target, half-life, serum clearance, etc.
The preclinical testing will allow researchers to allometrically estimate a safe starting dose of the antibody for clinical trials in humans. It will also allow to determine potential adverse effects. Based on pre-clinical trials, No Observable Effect Levels (NOEL) on drugs are established, which are used to determine initial phase 1 clinical trial dosage levels on a mass active pharmaceutical ingredient (API) per mass patient basis.

Preferably, preclinical testing includes the determination of the preclinical activity of the therapeutically effective antibody. The preclinical activity includes the testing of the therapeutically effective antibody's anti-tumor activity, anti-metastatic activity, tumor growth inhibition and/or inhibition of metastasis, preferably it includes the testing of tumor growth inhibition. Tumor growth inhibition is indicated in % inhibition occurring in a xenograft tumor model which is administered the therapeutically effective antibody in comparison to a xenograft tumor model which is not administered the therapeutically effective antibody, for example, 10, 20, 30, 40, 50, 60, 70, 80, 90 or 100% tumor growth inhibition (TGI); see the Figures and Examples.

"Detecting" when used herein refers to any technique or process in connection with near-infrared fluorescence imaging including fluorescence imaging and/or scanning technology used to detect signals emitted from a fluorescence label, for example, from a fluorescence label of the antibody described herein. It includes creating images of the region of the xenograft tumor model's body (or parts thereof), where the fluorescence signal is expected to be localizable. By way of example, if a fluorescence labeled antibody is expected to bind to the surface of malignant cells of the xenograft tumor, images are created from the region of the body where the xenograft tumor is located. Detecting includes the quantitative and/or qualitative analysis of the signals emitted from a fluorescence label, for example, from a fluorescence label of the antibody described herein.

Near-infrared fluorescence imaging which is preferred when detecting the fluorescence labelled antibodies applied in the methods of the present invention is a spectroscopic method which uses the near infrared region of the electromagnetic spectrum. In a preferred embodiment, near-infrared fluorescence imaging includes fluorescence reflectance imaging (FRI) or fluorescence-mediated tomography (FMT).

A "region" includes any part of the xenograft tumor model's body, any organ of the xenograft tumor model and the surface of the xenograft tumor model's body including skin, muscles and bones. "Organ" includes one or more organs selected from the the digestive system (including salivary glands, esophagus, stomach, liver, gallbladder, pancreas, intestines, rectum and anus); endocrine system (including endocrine glands such as the hypothalamus, pituitary or pituitary gland, pineal body or pineal gland, thyroid, parathyroids and adrenals, i.e., adrenal glands); integumentary system (including skin, hair and nails); lymphatic system (including lymphatic system, lymph nodes, tonsils, adenoids, thymus and spleen); muscular system; nervous system (including brain, spinal cord, peripheral nerves and nerves); reproductive system (including ovaries, fallopian tubes, uterus, vagina, mammary glands, testes, vas deferens, seminal vesicles, prostate and penis); respiratory system (including the pharynx, larynx, trachea, bronchi, lungs and diaphragm); skeletal system (including bones, cartilage, ligaments and tendons); and the urinary system (including kidneys, ureters, bladder and urethra involved in fluid balance, electrolyte balance and excretion of urine).

Likewise, it is envisaged that images are created from the region as described herein and/or from further regions of the xenograft tumor model. Specifically, it may be of interest to create images from the entire body of the xenograft tumor model to localize the fluorescence labelled antibody. For example, it may be of interest to monitor the binding and/or presence of the antibody in the blood to, for example, determine the binding specificity of the antibody and/or its distribution, half-time, etc. in order to aid in the selection of a candidate for the therapeutically most effective antibody out of a panel of potentially therapeutic effective antibodies.

"From further regions of the xenograft tumor model" means further defined or discrete parts, or regions of the xenograft tumor model, which might be of interest for any kind of measurement. For example, it is envisaged that the organ distribution and/or accumulation and/or secretion (determination of the secretion pathway) and/or metabolism (for example the generation of metabolites of a drug) of a fluorescence labeled antibody is to be detected and/or evaluated, which will for example aid in the determination of the most qualified xenograft tumor model out of a panel of potential xenograft tumor models. "Further regions" therefore comprises for example a part of an organ, an organ, blood vessel networks or nervous cell system of the xenograft tumor model. The created images can then be qualitatively and/or quantitatively analyzed. In the context of the method of the present invention, the created images are analyzed to determine the (fluorescence) signals from the fluorescence labeled antibody after binding to its target in said xenograft tumor model in order to select the most qualified xenograft tumor model on the basis of the height (intensity) and/or amount of the (fluorescence) signals.

Specifically, the xenograft tumor model which shows after binding of the therapeutically effective antibody to its target in the xenograft tumor model the highest fluorescence signals is preferably a candidate for the most qualified xenograft tumor model (out of a panel of potential xenograft tumor models) (selection step). More specifically, the height/intensity (i.e. quantitative amount) of the fluorescence signals of the antibody after binding to its target in the xenograft tumor of the xenograft tumor model is determined and compared to the height of fluorescence signals of the same antibody in another xenograft tumor model (i.e., relatively to each other). Accordingly, the height of the signal of the tested antibodies is determined and compared to each other, whereby the highest signal can be determined. Optionally, a background value (i.e. the height of the fluorescence signal emitted from the fluorescence labelled antibody when it is located elsewhere in the body of the xenograft tumor model, but not at the desired target) may be subtracted from the height of the fluorescence signal of each antibody (i.e. each fluorescence signal is normalize) before the highest signal is determined.

When referred to herein, the term "candidate for the most qualified xenograft tumor model" means that the xenograft tumor model is a prospective xenograft model that is potentially useful (i.e., most qualified) for preclinical testing of a therapeutically effective antibody. Such a candidate is deemed to show or shows preferably improved properties for preclinical testing of a therapeutically effective antibody in comparison to other xenograft tumor models. Preferably, said candidate is in accordance with the methods of the present invention selected out of a panel of potential qualified xenograft tumor models on the basis that it shows after a therapeutically effective antibody bound to its target on the surface of the xenograft tumor in vivo the highest fluorescence signals.

The term "most qualified" when used herein means that the xenograft tumor model is potentially most qualified for preclinical testing of a therapeutically effective antibody. "Potentially" means that the xenograft tumor model is a xenograft tumor model that has a likelihood or probability to be of high value (i.e., has sufficient suitability) for preclinical testing of a therapeutically effective antibody. Accordingly, the term "potential" when used in the context of the xenograft tumor models that can be selected in accordance with the methods of the present invention means that - though a xenograft tumor model is deemed to be most qualified - said xenograft tumor model does not necessarily have to be most qualified. However, the methods of the present invention aid in selecting the most qualified xenograft tumor model (out of a panel of potentially qualified xenograft tumor models).

Accordingly, the term "most qualified" preferably means that the xenograft tumor model selected in accordance with the methods and uses of the present invention shows after binding of the therapeutically effective antibody to its target in the xenograft tumor model the highest fluorescence signals in comparison to other xenograft tumor models out of a panel of xenograft tumor models.

A qualitative analysis of the created images is also envisaged, but less preferred, since the method of the present invention is for selecting the most qualified xenograft tumor model for the preclinical testing of a therapeutically effective antibody, whereby the most qualified xenograft tumor model can be selected on the basis of the highest fluorescence signals. In fact, the inventors observed that the xenograft tumor which shows the highest fluorescence signal after binding of the therapeutically active antibody to its target is such a most qualified xenograft tumor model.

As used herein, the term "binds" in all its grammatical forms when used in connection with the interaction between a desired target and the antibody to be applied in accordance with the present invention indicates that the antibody's epitope binding domain (i.e. the CDRs of the variable light and heavy chains (in case of domain antibodies only the CDRs of either the light or heavy chain), optionally in combination with one or more amino acids from the framework regions) associates with (e.g., interacts with or complexes with) the target to an antibody-antigen complex in a statistically significant degree as compared to association with proteins generally (i.e., non-specific binding). The term "epitope binding domain" is also understood to refer to a domain that has a statistically significant association or binding with a target.

Preferably, the method of the present invention aids in the in vivo selection of a xenograft tumor model most qualified for preclinical testing of a therapeutically effective antibody. The term "aiding" is used to indicate that the method according to the present invention will (together with other methods and/or variables, e.g., methods for selecting a xenograft tumor model which is most qualified for preclinical testing of a therapeutically effective antibody) aid the skilled artisan to select a xenograft tumor model which is most qualified for preclinical testing of a therapeutically effective antibody against a desired target on the surface of a malignant/cancerous cell. Put it differently, said other methods will help to confirm the selection of the most qualified xenograft tumor model. Examples of said other methods are immunochemistry, immunohistochemistry, histological techniques, for example, issue staining, or mRNA/PCR applications.

Specifically, for tissue staining, tumors of a subject, preferably a test animal such as a mouse, rat or monkey are explanted, sections are prepared, fixed, embedded in paraffin and stained with, for example, hematoxylin-eosin (HE). So-stained tumor sections can be imaged *ex vivo* by, for example, the Nuance® system.
As an example for tumor explantation, test animals are sacrificed and tumors are explanted according to committed guidelines (GVSolas, Felasa, TierschG). After sacrificing the animals, tumors are explanted using, for example, a scalpel and transferred into an embedding cassette or flash frozen in liquid nitrogen with isopentan for immunohistochemical applications and without isopentan for mRNA/PCR applications.

As an example for fixation, explanted tumors are enclosed in an embedding cassette and are incubated under continuous agitation in formalin for preferably approximately 24 hours. Thereafter, formalin is discarded and tumors washed with dest. water followed by dehydration of the tumors and penetration of paraffin. All the incubation steps are carried out using the Tissue Tek® VIP Vacuum Infiltration Processor.
After paraffin penetration, tumors are embedded in liquid paraffin with the Tissue Tek® Paraffin embedding station to a final histological block. Paraffin sections in a range of 2-8 µm of thickness are obtained from these blocks using a microtome. They are cut, uptaken on glass slides, air dried over night at 37 °C followed by de-paraffinization and HE-staining. Slides are examined with the Nuance system.

As regards immunohistochemistry, an unlabeled antibody is parenterally injected, preferably intravenously, tumors are explanted, preferably at around 24 hours post-injection of the antibody, flash frozen in, for example, liquid nitrogen and isopentan and sectioned. Unlabeled antibody is identified by using a secondary antibody directed against the Ig subtype of the unlabeled antibody, for example, goat anti-mouse IgG.

For Immunohistochemistry applications, tumors are flash frozen immediately after excision in Isopentan and liquid nitrogen and held at -20 °C until sectioning. For example, 10 µm tumor cryosections are cut at the optimum cutting temperature of -18 C with the Cryostat (2800 Frigocut), fixed with acetone for one minute, air dried and used for Immunohistochemistry application.

With respect to mRNA techniques, tumors are explanted and flash frozen in, for example, liquid nitrogen (without isopentan). Tumors are lysed, homogenized and RNA is prepared according to methods commonly known in the art. Afterwards, cDNA is prepared and analyzed qualitatively and/or quantitatively for the expression of a gene of interest, for example, that encoding the target on the surface of a mammalian cell against which an antibody is selected in accordance with the teaching of the present invention. As a loading control in a quantitative expression analysis a typical housekeeping gene such as β-actin or GAPDH can be used.

These other method will be one of the components taken into consideration by the skilled artisan helping i.e. aiding him to select a xenograft tumor model which is most qualified for preclinical testing of a therapeutically effective antibody.
The term "therapeutically effective" or "therapeutic efficacy" or any other grammatical form thereof refers to the capability of an antibody to "treat" a disease or disorder in a subject.

"Therapeutically effective" when used in the context of the antibodies that are to be preclinically tested in the xenograft tumor model selected in accordance with the methods and uses of the present invention includes that - though such an antibody is deemed to have a therapeutic effect which, for example, may have been tested with means and methods known in the art - said antibody does not necessarily have to be therapeutically effective in, for example, a subject, preferably a human. Thus, a therapeutically effective antibody is then first of all a candidate for the therapeutically most effective antibody.
However, preferably when used in the present invention, a "therapeutically effective" antibody is deemed to have shown a therapeutically effect in vitro and/or in vivo in tests commonly applied in the art before it is applied in the methods and uses of the present invention.

A "candidate for the therapeutically most effective antibody" is an antibody that is a prospective antibody that may be useful in an antibody-based therapy. Such a candidate shows preferably improved properties in vivo (such as binding, target recognition, affinity) in comparison to other potentially therapeutic effective antibodies. Preferably, said candidate is in accordance with the methods of the present invention selected out of a panel of potentially therapeutic effective antibodies on the basis that it shows after binding to its target on the surface of a mammalian cell in vivo the highest fluorescence signals.

In the case of cancer, the therapeutically effective amount of the antibody may reduce the number of cancer cells; reduce the tumor size; inhibit (i.e., slow to some extent and preferably stop) cancer cell infiltration into peripheral organs; inhibit (i. e., slow to some extent and preferably stop) tumor metastasis; inhibit, to some extent, tumor growth; and/or relieve to some extent one or more of the symptoms associated with the cancer.

The therapeutic effect which an antibody may have can be detected by all established methods and approaches which will indicate a therapeutic effect. It is, for example, envisaged that the therapeutic effect is detected by way of surgical resection or biopsy of an affected tissue/organ which is subsequently analyzed by way of immunohistochemical (IHC) or comparable immunological techniques. Alternatively it is also envisaged that the tumor markers in the serum of the patient (if present) are detected in order to diagnose whether the therapeutic approach is already effective or not. Additionally or alternatively it is also possible to evaluate the general appearance of the respective patient (fitness, well-being, decrease of tumor-mediated ailment etc.) which will also aid the skilled practitioner to evaluate whether a therapeutic effect is already there. The skilled person is aware of numerous other ways which will enable him or her to observe a therapeutic effect. For cancer therapy, therapeutic efficacy can be measured, for example, by assessing the time to disease progression (TTP) and/or determining the response rate (RR).

An imaging system useful in the practice of this invention typically includes three basic components: (1) excitation light, (2) a means for separating or distinguishing excitation light and emission light (preferably a software and/or hardware filter(s) which might be fitted to the excitation light and/or to the detection system), and (3) a detection system for receiving the light emitted from at least one fluorescent label and/or from the fluorescent entity and/or from the fluorescent analyte of the invention (optical detector). It is envisaged that the light source (excitation means) may optionally (i) comprise a pre-determined or tunable filter. The light source can be a suitably filtered white light, i.e., bandpass light from a broadband source. For example, light from a 150-watt halogen lamp can be passed through a suitable bandpass filter. In some embodiments, the light source is a laser. See, e.g., Boas et al., 1994, Proc. Natl. Acad. Sci. USA 91:4887-4891; Ntziachristos et al., 2000, Proc. Natl. Acad. Sci. USA 97:2767-2772; Alexander, 1991, J. Clin. Laser Med. Surg. 9:416-418. Information on near infrared lasers for imaging can be found at http://www.imds.com and various other well-known sources. A high pass or bandpass filter (e.g. 700 nm) can be used to separate optical emissions (emission light) from excitation light. Any suitable light detection/image recording component (an optical detector), e.g., charge coupled device (CCD) systems, a photodiode, a photoconductive cell, a complementary metal oxide semiconductor (CMOS) or photomultiplier tubes can be used in the invention. Said components are explained in more detail herein below. The choice of light detection/image recording will depend on factors including type of light gathering/image forming component being used. Selecting suitable components, assembling them into an imaging system of the invention, and operating the system is within ordinary skill in the art.

The in vivo imaging techniques include the FMT technology (fluorescence molecular tomography), a laser based three-dimensional imaging system, provides non-invasive, whole body, deep tissue imaging in small animal models and generates 3D reconstruction of fluorescence sources and/or allows measurement of fluorescence concentrations of fluorescence labeled structures due to binding of the fluorescence labeled antibody to its desired target as described herein. The system allows determining the real distribution of the fluorescence labeled antibody in a planar, two-dimensional imaging mode and/or 3D imaging studies with the FMT system allow improved assessment of the antibody distribution in the subject's body) Therefore, quantification of fluorescence signal intensities can be analyzed in a more realistic approach.
An example of a near-infrared fluorescence imaging system is the MAESTRO system described in the appended Examples. Accordingly, the MAESTRO system is a preferred system that may be applied in methods and uses of the present invention.

The MAESTRO system is a planar fluorescence-reflecting-imaging system that allows a non-invasive *in vivo* fluorescence measurement. In this multispectral analysis, a series of images are captured, at specific wavelengths. The range of wavelengths captured should cover the expected spectral emission range of the label present in the specimen. The result will be a series of images called "image cube" and it is the data within this series of images that is used to define the individual spectra of both auto-fluorescence and specific labels. Many labels of biological interest have emission spectra that are so similar that separation using expensive narrow band filters is difficult or impossible. A single long pass emission filter replaces a large collection of emission filters. In addition to the natural auto-fluorescence of the skin, fur, sebaceous glands, there is also distinct auto-fluorescence from commensal organisms (fungi, mites, etc.) and ingested food (chlorophyll). Multispectral analysis is able to separate all of these signals from the specific label applied to the specimen through the mathematically disentanglement of the linear signal mixture (unmixing) of the emitted fluorescent lights as long as the emission spectrum of the desired signal and of the auto-fluorescence are known.

Measurement with the MAESTRO system works as follows: The illumination module is equipped with a xenon lamp (Cermax) that excites white light. Through a downstream connected excitation filter (chosen by the experimenter), the light is delimitated to a, for the experiment, desired wavelength range and conducted via an optical fiber into the imaging module. In here, the restricted light is partitioned into four optical fibers that illuminate the anesthetized test animal. The MAESTRO system chooses the optimal exposure time automatically, so that there is no risk of overexposure. The emitted fluorescence light of the activated fluorescent probe is selected with an emission filter (see Table 1) and conducted through a liquid crystal (LC) to a high sensitive, cooled CCD-camera. The liquid crystal enables the camera a selective picture recording of a specific wavelength. The wavelength measurement range depends on the selected filter set (blue, green, yellow, red, deep red, NIR) and pictures are recorded in steps of 10 nm. The spectral information of each single picture is combined in one "picture package" that is called "image cube".

**Table 1: Maestro filter sets.**

| Maestro Filter Set | Part # | Excitation Filter | Emission Filter | Acquisition Settings* |
|---|---|---|---|---|
| Blue | M-MSI-FLTR-BLUE | 445 to 490nmm | 515 nm longpass | 500 to 720 in 10nm steps |
| Green | M-MSI-FLTR-GREEN | 503 to 555 nm | 580 nm longpass | 550 to 800 in 10nm steps |
| Yellow | M-MSI-FLTR-YELLOW | 575 to 605 nm | 645 nm longpass | 630 to 850 in 10nm steps |
| Red | M-MSI-FLTR-RED | 615 to 665 nm | 700 nm longpass | 680 to 950 in 10nm steps |
| Deep Red | M-MSI-FLTR-DEEP-RED | 671 to 705 nm | 750 nm longpass | 730 to 950 in 10nm steps |
| NIR | M-MSI-FLTR-NIR | 710 to 760 nm | 800 nm longpass | 780 to 950 in 10nm steps |

The analysis with the MAESTRO system works as follows:
Each recording compose of 12 bit black-and-white pictures that can be illustrated in 4096 different gray scales and therefore it is possible to discriminate between smallest differences in emission intensities. In contrast, the human eye is able to distinguish between 30-35 grey scales. Those values for the emission intensities (grey scales) are plotted against the wavelength range and as a result, we obtain the emission spectra of each probe and the tissue auto-fluorescence. The software subdivides the three fundamental colors (red, green, blue) to the wavelength range used for the imaging cube whereby the black-and-white pictures turn into colored image. Out of these acquired multispectral information the system is able to differentiate between injected probes and auto-fluorescence of any source. The program is using a spectral library, where the single spectra of each pure probe and the spectra acquired by imaging the study animals (Balbc/nude or Scid Beige mice) without any injection (mouse auto-fluorescence). By knowing the exact spectra of the pure imaging and of the auto-fluorescence, the system is able to filter the whole image for the desired spectra and assign a color to each of them. The originated image (unmixed composite image) shows the present spectra in different colors. To visualize the intensity distribution of the probe signal, it is possible to illustrate the signal in false colors, whereas low intensities are blue and regions of high intensities are red. Besides that, one can define a detection limit for the signal intensity of the probe, which allows reducing the signal of circulating probes and unspecific bindings.

Comparison and quantification with the MAESTRO system works as follows:
The MAESTRO's ability to compare fluorophore regions of an image makes it easy to compare the tumor fluorescent signal intensities during therapy. The program provides tools for the comparison of different signal intensities in tumor regions (compared images). Since all images are taken at optimal exposure times, they differ depending on the strength of signals. For a reliable comparison, the pictures are standardized to one exposure time, resulting in an illustration of differences in signal intensities. By manually drawing and modifying measurement regions, signal intensities can be quantified in intensity values. Once a measurement area is selected around the tumor, it can be cloned and moved to the next image to be compared with. Each region is calculated in pixels and mm² based on the current settings (stage height and binning). As a result, it gives information about the average signal, total signal, max. signal and average signal/exposure time (1/ms) within the created measurement area.

Another imaging technique which may be applied in connection with the present invention is the FMT technology (fluorescence molecular tomography), a laser based three-dimensional imaging system, which provides non-invasive, whole body, deep tissue imaging in small animal models and generates 3D reconstruction of fluorescence sources and/or allows measurement of fluorescence labeled analytes. The FMT technology is described, for example, in US 6,615,063.

When performing the methods and uses of the invention, it is preferred that the fluorescence labeled potentially therapeutic antibody is to be administered to the xenograft tumor model prior to detecting the antibody, i.e., the signals from the fluorescence labeled antibody.
Preferably, the antibody is administered parenterally, preferably intravenously to the xenograft tumor model.

The term "administering" in all of its grammatical forms means administration of a therapeutic effective antibody (preferably in the form of a pharmaceutical composition). If a panel of therapeutically effective antibodies is administered, the antibodies of said panel are either individually administered or as a panel, i.e., altogether, with individual administration being preferred.
If administered altogether, each antibody comprises preferably a different fluorescence label as described herein in order to identify each of the antibodies.

The term "antibody" refers to a monoclonal or a polyclonal antibody (see Harlow and Lane, "Antibodies, A Laboratory Manual", CSH Press, Cold Spring Harbor, USA, 1988) which binds to a target, or a derivative of said antibody which retains or essentially retains its binding specificity. Preferred derivatives of such antibodies are chimeric antibodies comprising, for example, a mouse or rat variable region and a human constant region. The antibody may be in an isolated form. The term "functional fragment" as used herein refers to fragments of the antibodies as specified herein which retain or essentially retain the binding specificity of the antibodies like, separated light and heavy chains, Fab, Fab/c, Fv, Fab', F(ab')2. The term "antibody" also comprises bifunctional (bispecific) antibodies and antibody constructs, like single-chain Fvs (scFv) or antibody-fusion proteins. The term "scFv fragment" (single-chain Fv fragment) is well understood in the art and preferred due to its small size and the possibility to produce such fragments recombinantly. Said antibody or antibody binding portion is a human antibody or a humanized antibody. The term "humanized antibody" means, in accordance with the present invention, an antibody of non-human origin, where at least one complementarity determining region (CDR) in the variable regions such as the CDR3 and preferably all 6 CDRs have been replaced by CDRs of an antibody of human origin having a desired specificity. Optionally, the non-human constant region(s) of the antibody has/have been replaced by (a) constant region(s) of a human antibody. Methods for the production of humanized antibodies are described in, e.g., EP-A1 0 239 400 and WO90/07861. The term antibody or functional fragment thereof also includes heavy chain antibodies and the variable domains thereof, which are mentioned in WO 94/04678, WO 96/34103 and WO 97/49805, WO 04/062551, WO 04/041863, WO 04/041865, WO 04/041862 and WO 04/041867; as well as domain antibodies or "dAb's", which are based on or derived from the heavy chain variable domain (VH) or the light chain variable domain (VL) of traditional 4 chain antibody molecules (see, e.g., Ward et al. 1989 Nature 341, 544-546).

An "isolated antibody" is one that has been identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. Preferably, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or non-reducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

There are five classes of immunoglobulins: IgA, IgD, IgE, IgG, and IgM, having heavy chains designated a, 8, s, y and, respectively. The y and a classes are further divided into subclasses on the basis of relatively minor differences in CH sequence and function, e. g., humans express the following subclasses: IgGI, IgG2, IgG3, IgG4, IgAI, and IgA2.

The term "variable" refers to the fact that certain segments of the variable domains differ extensively in sequence among antibodies. The V domain mediates antigen binding and defines specificity of a particular antibody for its particular antigen. However, the variability is not evenly distributed across the 1-10-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a P-sheet configuration, connected by three hypervariable regions, which form loops connecting, and in some cases forming part of, the P-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e. g. around about residues 24-34 (LI), 5056 (L2) and 89-97 (L3) in the VL, and around about 1-35 (HI), 50-65 (H2) and 95-102 (113) in the VH; Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (LI), 50-52 (L2) and 91-96 (U) in the VL, and 26-32 (HI), 53-55 (1-12) and 96-101 (H3) in the VH; Chothia and Lesk J. Mol. Biol. 196: 901-917 (1987)).

The term "monoclonal antibody" as used herein refers to an antibody obtained from a population of substantially homogeneous antibodies, i. e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations, which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method.
The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain (s) is identical with or homologous to corresponding sequences in antibodies derived from another species or belonging to another antibody class or subclass, as well as fragments of such antibodies, so long as they exhibit the desired biological activity (see U. S. Patent No. 4,816, 567; and Morrison et al. , Proc. Natl. Acad. Sci. USA, 81: 6851-6855 (1984)). Chimeric antibodies of interest herein include "primatized" antibodies comprising variable domain antigen-binding sequences derived from a non-human primate (e. g. Old World Monkey, Ape etc), and human constant region sequences.

An "intact" antibody is one that comprises an antigen-binding site as well as a CL and at least heavy chain constant domains, CHI, CH2 and CH3. The constant domains may be native sequence constant domains (e. g. human native sequence constant domains) or amino acid sequence variant thereof. Preferably, the intact antibody has one or more effector functions.

An "antibody fragment" comprises a portion of an intact antibody, preferably the antigen binding or variable region of the intact antibody. Examples of antibody fragments include Fab, Fab', F (ab') 2, and Fv fragments; diabodies; linear antibodies (see US patent 5,641, 870, Example 2; Zapata et al., Protein Eng. 8 (10): 1057-1062 [1995]) ; single-chain antibody molecules; and multispecific antibodies formed from antibody fragments. Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i. e. , it has a single antigen- binding site. Pepsin treatment of an antibody yields a single large F (ab') 2 fragment which roughly corresponds to two disulfide linked Fab fragments having divalent antigen- binding activity and is still capable of cross-linking antigen. Fab' fragments differ from Fab fragments by having additional few residues at the carboxy terminus of the CH1 domain including one or more cysteines from the antibody hinge region. Fab'-SH is the designation herein for Fab' in which the cysteine residue (s) of the constant domains bear a free thiol group. F(ab') 2 antibody fragments originally were produced as pairs of 8 Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, which region is also the part recognized by Fc receptors (FcR) found on certain types of cells.

"Fv" is the minimum antibody fragment which contains a complete antigen- recognition and-binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site. "Single-chain Fv" also abbreviated as "sFv" or "scFv" are antibody fragments that comprise the VH and VL antibody domains connected into a single polypeptide chain.

Preferably, the sFv polypeptide further comprises a polypeptide linker between the VH and VL domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 5-10 residues) between the VH and VL domains such that inter-chain but not intra-chain pairing of the V domains is achieved, resulting in a bivalent fragment, i. e., fragment having two antigen-binding sites. Bispecific diabodies are heterodimers of two "crossover" sFv fragments in which the VH and VL domains of the two antibodies are present on different polypeptide chains. Diabodies are described more fully in, for example, EP 404,097; WO 93/11161; and Hollinger et al., Proc. Natl. Acad. Sci. USA, 90: 6444-6448 (1993).

"Humanized" forms of non-human (e.g., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al. , Nature 321: 522-525 (1986); Riechmann et al., Nature 332: 323-329 (1988); and Presta, Curr. Op. Struct. Biol. 2: 593- 596 (1992).

The antibody applied in the methods and uses of the present invention is labeled with a fluorescence label, preferably a fluorescence label which is detectable by near-infrared fluorescence imaging. Accordingly, when used in the context of the present invention, the term "fluorescence" includes fluorescence labels commonly known in the art or as described herein and also includes preferably near-infrared fluorescence labels, the latter being preferred in the context of the present invention.

A "fluorescent label" as used herein characterizes a molecule which comprises a fluorophore. A fluorophore, which is sometimes also termed fluorochrome, is a functional group in a molecule which will absorb energy of a specific wavelength and re-emit energy at a different wavelength. Said different wavelength, when compared to the said specific (predetermined) wavelength, is re-emitted with a wavelength which is distinguishable from the specific (predetermined) wavelength, for example it is re-emitted with a longer wavelength or with a shorter wave-length, however in the latter case with decreased intensity. The amount and wavelength of the emitted energy depends on both the fluorophore and the chemical environment of the fluorophore.

It is envisaged that the labeled antibody comprises more than one fluorescence or near-infrared fluorescence label, for example, 2, 3, 4, 5, 6, 7, 8, 9 or 10. Likewise, it is envisaged that 2, 3, 4, 5, 6, 7, 8, 9 or 10 different fluorescence or near-infrared fluorescence labeled antibodies are applied in the methods of the present invention.

Accordingly, it is envisaged that the fluorescent entity as used in the context of the present invention comprises just one sort of fluorescent labels or a mixture of at least two, three, four, five or even more different sorts of fluorescent labels. "Just one sort" means that the fluorescent labels contain one and the same fluorophore, while different sorts means that the different fluorescent labels comprise different fluorophores and therefore show different absorptions and/or emission characteristics. These "different" characteristics may be "unmixed" subsequently, e.g. by way of software aided evaluations. Means and methods to unmix the emission of more than one different fluorophore are well known to the skilled person.
The fluorescent label can be covalently and/or non-covalently linked to the spacer or to the second entity of the fluorescent analyte, using any suitable reactive group on the fluorescent label and a compatible functional group on the spacer or the second entity.

In the context of the present invention, said fluorescent label is preferably selected from the group comprising quantum dot agents, fluorescent proteins, fluorescent dyes, pH-sensitive fluorescent dyes, voltage sensitive fluorescent dyes and/or fluorescent labeled microspheres.
An example of a fluorescent label are "Quantum dot agents" or "Quantum dots", also known as nanocrystals, are a special class of materials known as semiconductors, which are crystals composed of periodic groups of II-VI, III-V, or IV-VI materials.
Another example is a "Fluorescent protein" which includes for example green fluorescent protein (GFP), CFP, YFP, BFP either enhanced or not. Further fluorescent proteins are described in Zhang, Nat Rev Mol Cell Biol. 2002, 12, pages 906-18 or in Giepmans, Science. 2006, 312, pages 217-24.

"Fluorescent dyes" includes all kinds of fluorescent labels including but not limited to, Fluorescein including all its derivatives like for example FITC; Rhodamine including all its derivatives such as tetramethylrhodamine (TAMRA) and its isothiocyanate derivative (TRITC), sulforhodamine 101 (and its sulfonyl chloride form Texas Red), Rhodamine Red, and other derivatives of rhodamine which include newer fluorophores such as Alexa 546, Alexa 555, Alexa 633, DyLight 549 and DyLight 633); Alexa Fluors (the Alexa Fluor family of fluorescent dyes is produced by Molecular Probes); DyLight Fluor which is a family of fluorescent dyes are produced by Dyomics, ATTO Dyes, which represent a series of fluorescent labels and dyes manufactured by ATTO-TEC GmbH in Siegen, WO/2007/067978 Japan); LaJolla Blue (Diatron, Miami, Fla.); indocyanine green (ICG) and its analogs (Licha et al., 1996, SPIE 2927:192-198; Ito et al., U.S. Pat. No. 5,968,479); indotricarbocyanine (ITC; WO 98/47538), and chelated lanthanide compounds. Fluorescent lanthanide metals include europium and terbium.

An antibody is preferably labeled with a fluorescence label detectable by NIRF, i.e. it is preferably labeled with a near-infrared (NIR) fluorescence label. NIR fluorescence labels with excitation and emission wavelengths in the near infrared spectrum are used, i.e., 640-1300 nm preferably 640-1200 nm, and more preferably 640-900 nm. Use of this portion of the electromagnetic spectrum maximizes tissue penetration and minimizes absorption by physiologically abundant absorbers such as hemoglobin (<650 nm) and water (>1200 nm). Ideal near infrared fluorochromes for *in vivo* use exhibit:
(1) narrow spectral characteristics,
(2) high sensitivity (quantum yield),
(3) biocompatibility,
(4) decoupled absorption and excitation spectra, and
(5) photo stability.

Various near infrared (NIR) fluorescence labels are commercially available and can be used to prepare a fluorescent entity according to this invention. Exemplary NIRF labels include the following: Cy5.5, Cy5 and Cy7 (Amersham, Arlington Hts., IL; IRD41 and IRD700 (LI-COR, Lincoln, NE); NIR-I, (Dejindo, Kumamoto, Japan); LaJolla Blue (Diatron, Miami, FL); indocyanine green (ICG) and its analogs (Licha, K., et al., SPIE-The International Society for Optical Engineering 1996; Vol. 2927: 192-198; US 5,968,479); indotricarbocyanine (ITC; WO 98/47538); and chelated lanthanide compounds and SF64, 5-29, 5-36 and 5-41 (from WO 2006/072580). Fluorescent lanthanide metals include europium and terbium. Fluorescence properties of lanthanides are described in Lackowicz, J. R., Principles of Fluorescence Spectroscopy, 2nd Ed., Kluwer Academic, New York, (1999).

"Fluorescent microspheres" are described in great detail in WO/2007/067978.

In a further embodiment of the present invention, at least one fluorescent label of the fluorescent entity is activatable. It is also envisaged that the fluorescent entity is activatable.

As mentioned before, it is known that the amount and wavelength of the energy emitted by a fluorescent dye depends on both the fluorophore and the chemical environment of the fluorophore. It follows that fluorescent dyes may react pH-sensitive or voltage sensitive, i.e. they are activatable by such changes in the chemical environment. Further activatable fluorescent labels are described for example in great detail in US 2006/0147378 A1, US 6592847, US 6,083,486, WO/2002/056670 or US 2003/0044353 A1.

By "activation" of a fluorescent label/entity is meant any change to the label/entity that alters a detectable property, e. g., an optical property, of the label/entity. This includes, but is not limited to, any modification, alteration, or binding (covalent or non-covalent) of the label/entity that results in a detectable difference in properties, e. g., optical properties e. g., changes in the fluorescence signal amplitude (e. g., dequenching and quenching), change in wavelength, fluorescence lifetime, spectral properties, or polarity. Optical properties include wavelengths, for example, in the visible, ultraviolet, near-infrared, and infrared regions of the electromagnetic spectrum. Activation can be, without limitation, by enzymatic cleavage, enzymatic conversion, phosphorylation or dephosphorylation, conformation change due to binding, enzyme-mediated splicing, enzyme-mediated transfer of the fluorophore, hybridization of complementary DNA or RNA, analyte binding such as association with an analyte such as Na+, K+, Ca2+, CI-, or another analyte, change in hydophobicity of the probe environment, and chemical modification of the fluorophore. Activation of the optical properties may or may not be accompanied by alterations in other detectable properties, such as (but not limited to) magnetic relaxation and bioluminescence.

Fluorescence labeling is accomplished using a chemically reactive derivative of a fluorophore. Common reactive groups include amine reactive isothiocyanate derivatives such as FITC and TRITC (derivatives of fluorescein and rhodamine), amine reactive succinimidyl esters such as NHS-fluorescein, and sulfhydryl reactive maleimide activated fluors such as fluorescein-5-maleimide. Reaction of any of these reactive dyes with another molecule results in a stable covalent bond formed between a fluorophore and a labeled molecule. Accordingly, the antibody used in the present invention can be labelled with the aforementioned fluorescence labels. Other suitable fluorescence labels envisaged by the present invention are Alexa Fluors, Dylight fluors and ATTO Dyes. Likewise, fluoresecent proteins such as GFP, YFP or CFP may be used. Also, activatable fluorescent dyes which are activated by pH changes or voltage, temperature are envisaged to be used. It is also envisaged that the labeled antibody comprises more than one fluorescence or near-infrared fluorescence label, for example, 2, 3, 4, 5, 6, 7, 8, 9 or 10. Likewise, it is envisaged that 2, 3, 4, 5, 6, 7, 8, 9 or 10 different fluorescence or near-infrared fluorescence labeled antibodies are applied in the methods and uses of the invention and the kits as described herein.

In the alternative, the antibody applied in the methods of the present invention may be indirectly labeled. For example, an unlabeled antibody is labeled (due to its binding to the constant region of the unlabeled antibody) by a second antibody comprising a fluorescence label, wherein said second antibody is directed against the unlabeled antibody.
In said alternative, the fluorescent label of the second antibody is activated once the epitope binding domain of said second antibody has bound to its target.

"Activated" includes the activation of activatable fluorescent labels which have been mentioned herein before. "Activated" also includes "FRET-based" effects. Forster resonance energy transfer (abbreviated FRET), also known as fluorescence resonance energy transfer, resonance energy transfer (RET) or electronic energy transfer (EET), is a mechanism describing energy transfer between two fluorophores. FRET provides an indication of proximity between donor and acceptor fluorophores. When a donor is excited with incident radiation at a defined frequency, some of the energy that the donor would normally emit as fluorescence is transferred to the acceptor, when the acceptor is in sufficiently close proximity to the donor (typically, within about 50 Angstroms for most donor fluorophores). At least some of the energy transferred to the acceptor is emitted as radiation at the fluorescence frequency of the acceptor. FRET is further described in various sources, such as "FRET Imaging" (Jares-Erijman, E.A, and Jovin, T.M, Nature Biotechnology, 21(11), (2003), pg 1387-1395. Screening systems based on such FRET effects are well known and described for example in WO 2006107864.

Methods for coupling fluorescent labels including NIR fluorescence labels are well known in the art. The conjugation techniques of these labels to an antibody have significantly matured during the past years and an excellent overview is given in Aslam, M., and Dent, A., Bioconjugation (1998) 216-363, London, and in the chapter "Macromolecule conjugation" in Tijssen, P., "Practice and theory of enzyme immunoassays" (1990), Elsevier, Amsterdam.
Appropriate coupling chemistries are known from the above cited literature (Aslam, supra). The fluorescent label, depending on which coupling moiety is present, can be reacted directly with the antibody either in an aqueous or an organic medium. The coupling moiety is a reactive group or activated group which is used for chemically coupling of the fluorochrome label to the antibody. The fluorochrome label can be either directly attached to the antibody or connected to the antibody via a spacer to form a NIR fluorescence label conjugate comprising the antibody and a NIR fluorescence label. The spacer used may be chosen or designed so as to have a suitably long *in vivo* persistence (half-life) inherently.

In a further aspect, the present invention relates to the use of the methods of the present invention for in vivo selecting a xenograft tumor model which is most qualified for preclinical testing of a therapeutically effective antibody.

In another aspect, the present invention relates to the use of near-infrared fluorescence imaging (NIRF) in a non-invasive method for in vivo selecting a xenograft tumor model which is most qualified for preclinical testing of a therapeutically effective antibody comprising
(a) detecting in one or more xenograft tumor models said therapeutically effective antibody which is fluorescence labeled after binding to its target in said xenograft tumor model by near-infrared fluorescence imaging (NIRF); and
(b) selecting the xenograft tumor model which shows after binding of said therapeutically effective antibody to said target in said xenograft tumor model the highest fluorescence signals.

The embodiments described in the context of the methods of the present invention are applicable in the context of the uses of the present invention, mutatis mutandis.

Also contemplated by the present invention is a xenograft tumor model selected by the method of any of the preceding embodiments for use in preclinical testing of a therapeutically effective antibody.

Further disclosed is a kit for in vivo selecting a xenograft tumor model comprising a therapeutically effective antibody, means for fluorescence labeling (as described herein) said antibody and one or more xenograft tumor models.

The embodiments described in the context of the methods of the present invention are applicable in the context of the kits described herein, mutatis mutandis.

Further described herein is a kit for preclinical testing of a therapeutically effective antibody comprising a therapeutically effective antibody, means for fluorescence labeling (as described herein) said antibody and one or more xenograft tumor models selected by the method of any of the preceding embodiments, preferably the most qualified xenograft tumor model.
Alternatively, rather than the xenograft tumor model, the kit can comprise one or more human tumor cell lines or primary human tumor cells or cell lines which can be used to generate a xenograft tumor model.

The embodiments described in the context of the methods of the present invention are applicable in the context of the kits described herein, mutatis mutandis.

In sum, the aspect that relates to the selection of a xenograft tumor model which is most qualified for preclinical testing of a therapeutically effective antibody can be summarized in the following items
(1) A non-invasive method of in vivo selecting a xenograft tumor model which is most qualified for preclinical testing of a therapeutically effective antibody
   (a) detecting in one or more xenograft tumor models said therapeutically effective antibody which is fluorescence labeled after binding to its target in said xenograft tumor model by near-infrared fluorescence imaging (NIRF); and
   (b) selecting the xenograft tumor model which shows after binding of said therapeutically effective antibody to said target in said xenograft tumor model the highest fluorescence signals.
(2) The method of item 1, wherein said xenograft tumor model is a mouse, rat, guinea pig, hamster or non-human monkey.
(3) The method of any of the preceding items, wherein said xenograft tumor model comprises a tumor.
(4) The method of item 3, wherein said tumor is from a human.
(5) The method of item 3 or 4, wherein said tumor comprises malignant/cancerous cells having a target for the therapeutically active antibody on their surface.
(6) The method of item 5, wherein said target is a protein having an extracellular portion.
(7) The method of any of the preceding items, wherein near-infrared fluorescence imaging includes fluorescence reflectance imaging (FRI) or fluorescence-mediated tomography (FMT).
(8) The method of any of the preceding items, wherein said xenograft tumor model is to be administered the fluorescence labeled therapeutically effective antibody prior to detecting said antibody.
(9) The method of any of the preceding items which is for in vivo selecting a xenograft tumor model which is most qualified for preclinical testing of a therapeutically effective antibody.
(10) Use of the method of any of the preceding items for in vivo selecting a xenograft tumor model which is most qualified for preclinical testing of a therapeutically effective antibody.
(11) Use of near-infrared fluorescence imaging (NIRF) in a non-invasive method for in vivo selecting a xenograft tumor model which is most qualified for preclinical testing of a therapeutically effective antibody comprising
   (a) detecting in one or more xenograft tumor models said therapeutically effective antibody which is fluorescence labeled after binding to its target in said xenograft tumor model by near-infrared fluorescence imaging (NIRF); and
   (b) selecting the xenograft tumor model which shows after binding of said therapeutically effective antibody to said target in said xenograft tumor model the highest fluorescence signals.
(12) A xenograft tumor model selected by the method of any of the preceding items for use in preclinical testing of a therapeutically effective antibody.
(13)A kit for in vivo selecting a xenograft tumor model comprising a therapeutically effective antibody, means for fluorescence labeling said antibody and one or more xenograft tumor models.
(14) A kit for preclinical testing of a therapeutically effective antibody comprising a therapeutically effective antibody, means for fluorescence labeling said antibody and one or more xenograft tumor models by the method of any of the preceding items.

The above findings are likewise applicable to the stratification of human patients as will be explained in the following in detail.

The embodiments/definitions described in the context of the xenograft tumor model above, apply, if not stated otherwise, mutatis mutandis to the now following embodiments.

In a further aspect, the present invention relates to a non-invasive method for identifying a cancer--patient disposed to respond favorably to a therapeutic antibody, which method comprises:
(a) directing excitation light of at least one predetermined wavelength onto a predetermined region of said patient,
(b) receiving emission light emitted from a fluorescently labeled therapeutic antibody with a wavelength distinguishable from the predetermined wavelength of (a), thereby identifying a cancer patient,
wherein said cancer--patient is one who had received said therapeutic antibody in a fluorescently labeled form either prior to the identification, prior to step (a) or prior to step (b), and wherein the method further comprises detecting, ex vivo, the fluorescently labeled antibody and thereby a subset of tumor cells in a tissue sample from the patient by direct or indirect immunohistochemistry, wherein said patient is one who had received the fluorescently labeled antibody prior to the removal of said tissue sample,
thereby identifying a cancer-patient whose tumor cells are labeled by said antibody, said patient being disposed to respond favorably to said therapeutic antibody.

It is preferred that said therapeutic antibody is to be administered to said patient in a therapeutically effective amount, i.e. said patient has received said labeled antibody in a therapeutically effective amount. By "therapeutically effective amount" or "therapeutically active" is meant a dose of an therapeutic antibody that produces the therapeutic effects for which it is administered.

In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. The exact dose will depend on the purpose of the treatment, and will be ascertainable by one skilled in the art using known techniques. As is known in the art and described above, adjustments for age, body weight, general health, sex, diet, drug interaction and the severity of the condition may be necessary, and will be ascertainable with routine experimentation by those skilled in the art. The therapeutic effect of the respective methods or method steps of the present invention is detectable as described herein.

It will be understood that the excitation light used in step (a) above is theoretically able to excite the label of said therapeutic antibody.

"Non-invasive" as used herein means that the methods, uses of the invention and/or kits/devices as described herein do not create skin and/or mucosa breaks of said subject, but do allow and involve contact of the skin/mucosa, with radiation and, likewise, penetration of the skin/&mucosa and all subsequent layers by radiation. Radiation thereby includes all kinds of light such as e.g. excitation light, emission light etc. which is described herein in the context of the present invention.

The methods/uses of the present invention and kits as described herein may be used for the identification of patients disposed to respond favorably to said therapeutic antibody. The term "identifying a patient disposed to respond favorably to a therapeutic antibody" includes stratifying a patient. The term "stratifying" refers to sorting patients into those who may or may not benefit from anti-tumor therapy with said therapeutic antibody. In particular, stratifying patients involves determining the emission light in accordance with the teaching described herein thereby deciding whether the patient may benefit from said therapeutic antibody or not. Those patients whose cancer/tumor cells are labeled by said fluorescently therapeutic antibody may benefit from said therapy, while those whose tumor cells are not labeled by said antibody may not benefit. To this end, it is envisaged to direct excitation light of at least one predetermined wavelength onto a predetermined region of said patient, wherein said predetermined region of said patient comprises said tumor/cancer cells or the tumor as such. In other words, the labeled therapeutic antibody binds to the mentioned tumor cells which results in a detectable signal in step (b) of the above mentioned method and the corresponding methods and uses disclosed herein. In that event, it is evident that the labeled therapeutic antibody may have a beneficial effect, i.e. the patient may respond favorably or benefits from the treatment. Provided that the antibody does not bind to the tumor of said patient, then there will be no or just an irrelevant signal (emission light) which as such already indicates that the therapeutic antibody, albeit it was thought that it could have a beneficial effect, is in fact not the best choice for that very patient. In order to be able to decide whether the signal obtained from the emission step in step (b) of the method above (and all corresponding methods disclosed herein), is relevant or irrelevant, it is envisaged to compare the obtained signal with a signal obtained with the very same labeled antibody in a further predetermined region of said patient, which further region is characterized in that it mainly contains healthy cells or tissue which is/are of the same source or origin (e.g. both are breast tissue or both are lung tissue etc.) as the tissue in question (in fact the tumor as such, parts of the tumor or the tumor cells). This comparison will aid the skilled person to define a suitable threshold level of fluorescence which might be regarded as negative (for example healthy breast tissue, preferably of the same patient might show some unspecific fluorescence which fluorescence is not predictive for the success of the therapy). Alternatively, it is also envisaged to compare the obtained data with reference data in a database or with reference data obtained with a different yet healthy patient ("healthy" in regard to the tumor/cancer).
It is preferred that said patient is one who has been diagnosed to suffer from cancer.

The term "tumor" as used herein in the context of the stratification methods refers to or describes the physiological condition in mammals that is typically characterized by unregulated cell growth. Examples of tumors include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, and melanoma. Solid tumors elected from the group of gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer, preferably breast cancer, are also included. Breast cancer is preferred.

It is also envisaged to employ the therapeutic antibodies of the present invention together with one or more additional anti-tumor agents. The term "anti-tumor agents" includes therapies which can be selected from antineoplastic agents, anti-angiogenic agents, chemotherapeutic agents and peptide cancer therapy agents. The antineoplastic agents can be selected from antibiotic-type agents, alkylating agents, antimetabolite agents, hormonal agents, immunological agents, interferon-type agents, kinase inhibitors, and combinations thereof. Such pharmaceutically active compounds/agents can be a traditional small organic chemical molecules or can be macromolecules such as a proteins, antibodies (including fragments thereof), peptibodies, DNA, RNA or fragments of such macromolecules.

As used herein, the term "excitation light" is used to describe light generated by an excitation light source. The excitation light includes, but is not limited to, spectral light components (i.e., wavelengths) capable of exciting fluorescence from a fluorophore. The spectral components in the excitation light that are capable of exciting fluorescent light can include a single wavelength, a single band of wavelengths, more than one wavelength, or more than one spectral band of wavelengths. The spectral components in the excitation light that are capable of exciting fluorescent light can include one or more wavelengths in the visible spectral regions of about 400 to 700 nanometres (nm). However, the spectral components in the excitation light that are capable of exciting fluorescent light can also include one or more wavelengths in the other spectral regions, for example, in the near infrared (NIR) spectral region of about 700 to 1000 nanometres, or in the ultra-violet (UV) spectral region of about 1 to 400 nanometres. The excitation light can further include spectral components that do not excite fluorescent light. The spectral components of the excitation light that are capable of exciting fluorescent light can have wavelengths shorter than the fluorescent light that they excite. However, in other arrangements, some additional spectral components of the excitation light can have wavelengths longer than the fluorescent light that they excite. In a preferred embodiment, the excitation light comprises a spectral band in the range of about 671 to 705 nm (ICG). The excitation light may be continuous in intensity, continued in wave, pulsed, or may be modulated (for example by frequency or amplitude) or any suitable combination thereof.

When the excitation light encounters a fluorescent label, the light is absorbed. Fluorescence occurs when the fluorescent label relaxes to its ground state after being excited. The fluorescent label then emits light that has detectably different (distinguishable) properties i.e., spectral properties - e.g. a slightly longer wavelength etc., from the excitation light. A part of the absorbed energy is transformed into heat. This loss of energy causes a wavelength shift from the shorter excitation wavelength to a longer emission wavelength. This process is known as the Stokes-Shift. However different optical phenomena like those described in Xu et al. (1996), Proc. Natl. Acad. Sci. 93: 10763-10768 can also be used to generate fluorescence.
In the context of the present invention, it is envisaged that excitation light comprising at least one, i.e. one, two, three, four, five or even more predetermined and preferably distinguished or distinguishable wavelength(s), is directed onto a predetermined (sometimes also denoted "delineated" region) region of the subject. A "delineated region" or "predetermined region" thereby encompasses, at most (maximal), the whole body of the subject or any smaller part of that body.
In a further preferred embodiment of the uses/methods/kits of the inventions embodiments, said predetermined region of said patient comprises at least the breast, liver, a kidney, bladder, lung, prostate, or pancreas of said patient.

It is also envisaged that in the methods of the invention, said excitation light of at least one predetermined wavelength is exclusively directed onto a delineated region of said subject. "Exclusively" means in this regard that said excitation light is at most (maximal) directed onto the mentioned delineated region, but not on any other part of the subject.

Defining a predetermined region and exciting light in order to produce a fluorescent signal as well as the detection of said signal are explained in great detail in WO2011/012646 (PCT/EP2010/060957).

The fluorescent label is preferably selected from the group consisting of quantum dot agents, fluorescent dyes, pH-sensitive fluorescent dyes, voltage sensitive fluorescent dyes, and fluorescent labeled microspheres.

........................................................................... The therapeutic antibody is preferably selected from the group consisting of alemtuzumab, apolizumab, cetuximab, epratuzumab, galiximab, gemtuzumab, ipilimumab, labetuzumab, panitumumab, rituximab, trastuzumab, nimotuzumab, mapatumumab, matuzumab, rhMab ICR62, rhMab B-Ly1 and pertuzumab, trastuzumab, and/or omnitarg, including combinations thereof.
It is envisaged that in step (b) said light with a wavelength distinguishable from the predetermined wavelength of (a) is received with an optical detector. The term "optical detector" has been defined herein elsewhere.
It is preferred that said subject has received said therapeutic antibody in a therapeutically effective dose prior to the identification.

The above methods further comprise the step of detecting, ex vivo, a subset of tumor cells in a tissue sample received from the tumor of said patient, wherein said patient is one who has received the fluorescently labeled antibody prior to the removal of said tissue sample.

A "tumor sample" (sometimes also denoted as "tissue sample") is preferably derived from a subject and may be obtained via biopsy such as needle biopsy, surgical biopsy, bone marrow biopsy etc. A tumor sample, thus, includes a tumor, parts of a tumor, tumor cells derived from a tumor (including tumor cell lines which may be derived from a tumor and which are grown in cell culture), but also tumor cell lines as such, and cells and/or tissue which are/is derived from a subject and which are/is suspected of being tumorigenic or even cancerous or which are/is suspected of comprising tumorigenic or cancerous cells. It is thus envisaged that the tumor sample may also comprise non-tumorigenic cells. For example tumor cells and/or (micro) metastases are frequently surrounded by healthy, i.e. non-tumorigenic tissue, i.e. the tumor cells could then form a subset of cells within the healthy tissue. A tumor sample thereby could comprise a subset of healthy (non-tumorigenic) cells and a subset of tumorigenic cells.

Said subset of cells is detected by direct or indirect immunohistochemistry (IHC).

The present disclosure further relates to an antibody for use in the treatment of a patient which has been diagnosed to respond favorably to said antibody with a method as defined in any one of the preceding claims.

### Figures

The figures show:
- **Figure 1.1:**: IHC staining of IGF1R in Calu3 lung cancer xenograft.
- **Figure 1.2:**: In vivo imaging of anti-IGF1R-Cy5 mAb in Calu3 lung cancer xenograft.
- **Figure 1.3:**: Therapeutic efficacy of anti-IGF1R mAb in Calu3 lung cancer xenograft.
- **Figure 2.1:**: IHC staining of IGF1R in A549 lung cancer xenograft.
- **Figure 2.2:**: In vivo imaging of anti-IGF1R-Cy5 mAb in A549 lung cancer xenograft.
- **Figure 2.3**:: Therapeutic efficacy of anti-IGF1R mAb in A549 lung cancer xenograft.
- **Figure 3.1:**: IHC staining of Her2 and Her3 in Calu3 lung cancer xenograft.
- **Figure 3.2:**: In vivo imaging of anti-HER2-Cy5 and anti-HER3-Cy5 mAb in Calu3 lung cancer xenograft.
- **Figure 3.3**:: Ex vivo imaging of anti-HER2-Cy5 and anti-HER3-Cy5 mAb in Calu3 lung cancer xenograft.
- **Figure 3.4:**: Therapeutic efficacy of anti-HER3 and anti-HER2 mAb in Calu3 lung cancer xenograft.
- **Figure 4.1**: In vivo imaging of anti-HER2-Cy5 and anti-IGF1R-Cy5 mAb in Calu3 lung cancer xenograft.
- **Figure 4.2**: Ex vivo imaging of anti-HER2-Cy5 and anti-IGF1R-Cy5 mAb in Calu3 lung cancer xenograft.

### Examples

The following examples illustrate the invention. The examples are included for purposes of illustration and the present invention is limited only by the claims.

### Example 1

The first example compares the ability of Immunohistochemistry (IHC) and near-infrared fluorescence imaging (NIRF), to verify the expression level of the tumor associated IGF1-Receptor's in Calu3 lung cancer xenograft.

For the detection of IGF1-Receptor by classic IHC, the Calu3 tumors are explanted form the animal and fixed in for 24 hours in formalin. Thereafter, the tumor tissue is dehydrated and embedded in paraffin. The tissue is cut, uptaken on glass slides, air dried over night at 37 °C followed by de-paraffinization and heat-induced epitope retrieval (Dako Target Retrieval Solution). For staining the IGF1-Receptor an anti-IGF1R mouse mAb form Calbiochem (Ab-4, CI24-31) is used and is performed as specified by the manufacturer. After that, the slides are covered with a cover glass and visualized by bright field microscopy. The percentage of positive cells and staining intensity are analyzed by an approved Pathologist and resulted in a high staining intensity of ++-+++ and 90% of positive cells. Thereby, IHC detection of IGF1-Receptor exhibit a high expression level in Calu3 lung cancer xenograft (**Figure 1.1**).

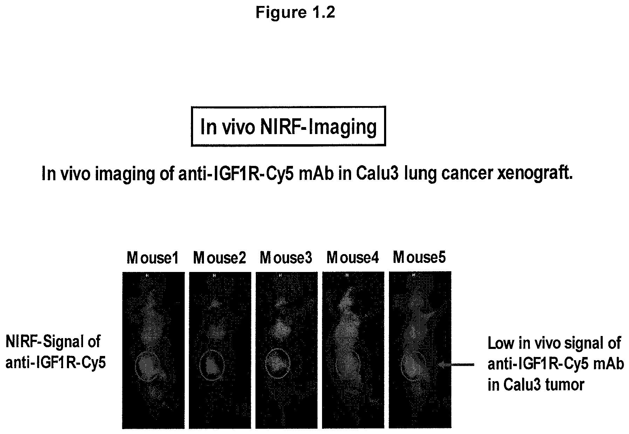

For non-invasive detection of the IGF1-Receptor by near-infrared fluorescence imaging, five s.c. Calu3 tumor bearing mice are injected with a Cy5 labeled anti-IGF1R mAb. After a single i.v. injection of 50 microgram of the labelled mAb, the fluorescence signal is imaged in the tumor area after 48 hours. The signals of all in vivo images are normalized and converted into pseudocolor. The imaging results show a very low signal intensity in the tumor region. It indicates that no or only a very low binding of antibody to the IGF1-Receptor take place **(****Figure 1.2****).**

The experimental proof of IGF1R in Calu3 by IHC and NIRF-Imaging lead to different results. IHC indicate a highly positive receptor expression and consequently Calu3 should be an appropriate preclinical xenograft model for testing the antitumor efficacy of anti-IGF1R mAb. In comparison the NIRF-Imaging of the therapeutic anti-IGF1R-Cy5 mAb show nearly no signal in the tumor area and would therefore not priories Calu3 for a preclinical study.

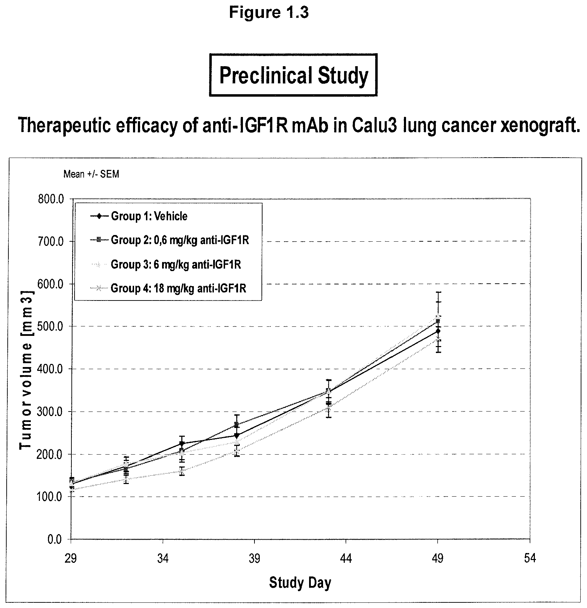

To confirm those previous data, the anti-IGF1R therapeutic antibody is tested in a preclinical study. The s.c. Calu3 bearing Balb/c nude mice are divided in four groups with ten mice each. The untreated vehicle group (1) are injected i.p. with Histidin buffer once a week. The treatment groups (2-4) are injected i.p. with a therapeutic anti-IGF1R antibody concentration of 0.6, 6 and 18 mg/kg once a week. The tumor volumes of all animals are measured over the time by calliper (**Figure 1****.****3**)**.**

The anti-IGF1R monoclonal antibody possesses no antitumor activity in Calu3 lung cancer xenografts and confirms therefore the results from NIRF-Imaging. The prioritization via IHC lead to a wrong result and failed.

### Example 2

The next example compares the ability of Immunohistochemistry (IHC) and near-infrared fluorescence imaging (NIRF), to verify the expression level of the tumor associated IGF1-Receptor's in A549 lung cancer xenograft.

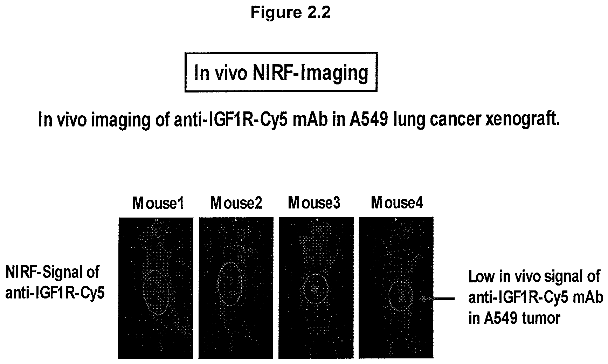

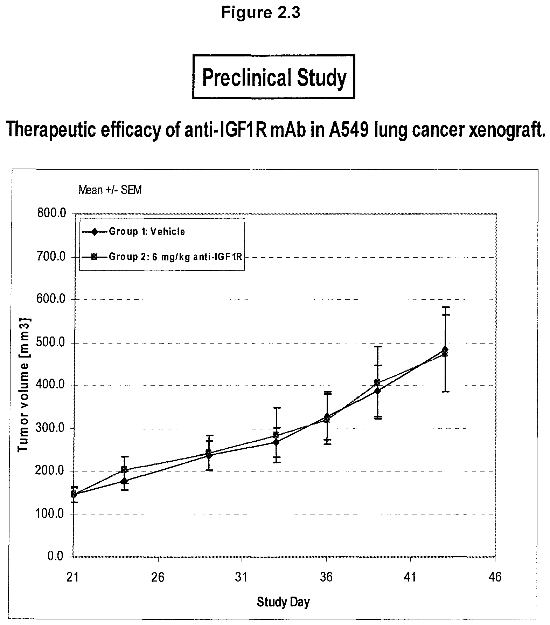

For the detection of IGF1-Receptor by classic IHC, the A549 tumors are explanted form the animal and fixed in for 24 hours in formalin. Thereafter, the tumor tissue is dehydrated and embedded in paraffin. The tissue is cut, uptaken on glass slides, air dried over night at 37 °C followed by de-paraffinization and heat-induced epitope retrieval (Dako Target Retrieval Solution). For staining the IGF1-Receptor an anti-IGF1R mouse mAb form Calbiochem (Ab-4, CI24-31) is used and is performed as specified by the manufacturer. After that, the slides are covered with a cover glass and visualized by bright field microscopy. The percentage of positive cells and staining intensity are analyzed by an approved Pathologist and resulted in a high staining intensity of ++-+++ and 80 to 90% of positive cells. Thereby, IHC detection of IGF1-Receptor exhibit a high receptor expression level in A549 lung cancer xenograft (**Figure 2****.****1**).

For non-invasive detection of the IGF1-Receptor by near-infrared fluorescence imaging, four s.c. A549 tumor bearing mice are injected with a Cy5 labeled anti-IGF1R mAb. After a single i.v. injection of 50 microgram of the labelled mAb, the fluorescence signal is imaged in the tumor area after 48 hours. The signals of all in vivo images are normalized and converted into pseudocolor. The imaging results show a very low signal intensity in the tumor region. It indicates that no or only a very low binding of antibody to the IGF1-Receptor take place (**Figure 2.2**).

The experimental proof of IGF1R in A549 by IHC and NIRF-Imaging lead to different results. IHC indicate a highly positive receptor expression and consequently A549 should be a appropriate preclinical xenograft model for testing the antitumor efficacy of anti-IGF1R mAb. In comparison the NIRF-Imaging of the therapeutic anti-IGF1R-Cy5 mAb show a nearly no signal in the tumor area and would therefore not priories A549 for a preclinical study.

To confirm those previous data, the anti-IGF1R therapeutic antibody is tested in a preclinical study. The s.c. A549 bearing Balb/c nude mice are divided in two groups with ten mice each. The untreated vehicle group (1) are injected i.p. with Histidin buffer once a week and the treatment group (2) are injected i.p. with a therapeutic anti-IGF1R antibody concentration of 6 mg/kg once a week. The tumor volume of all animals are measured over the time by calliper (**Figure 2****.****3**).

The anti-IGF1R monoclonal antibody possesses no antitumor activity in A549 lung cancer xenografts and confirms therefore the results from NIRF-Imaging. The prioritization via IHC lead to a wrong result and failed.

### Example 3

The next example compares the ability of Immunohistochemistry (IHC) and near-infrared fluorescence imaging (NIRF), to verify the expression level of the tumor associated HER2- and HER3-Receptor's in Calu3 lung cancer xenograft.

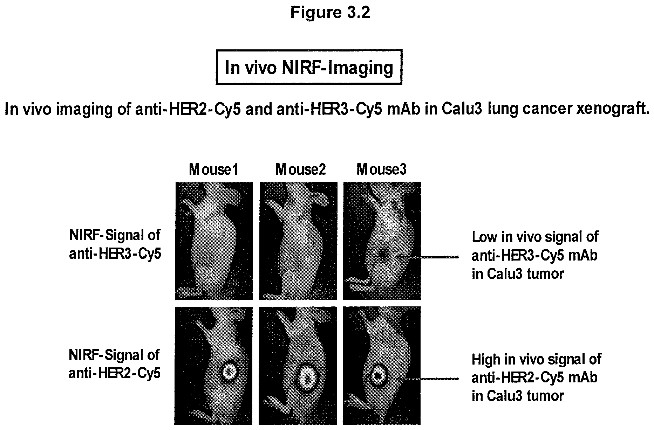

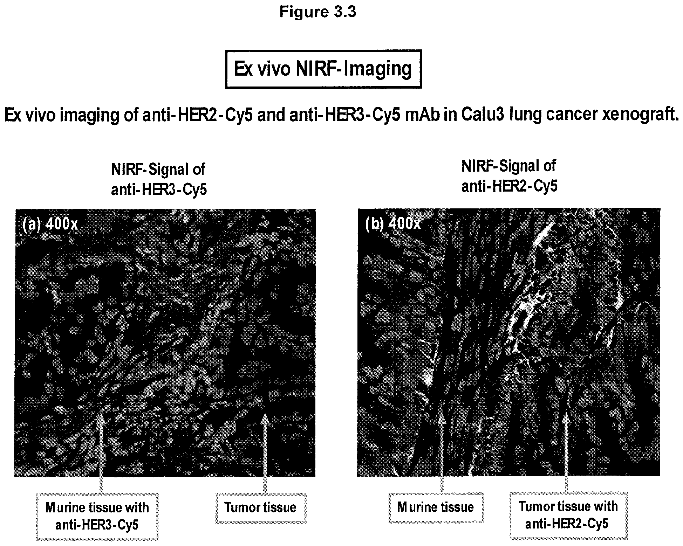

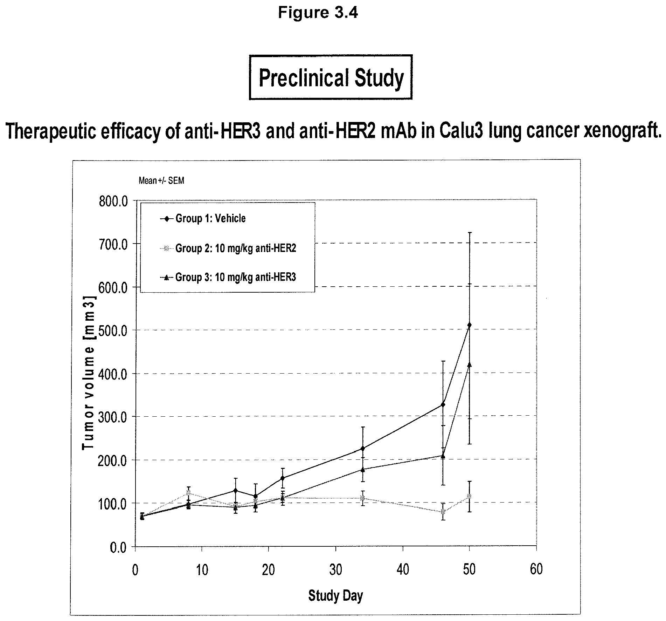

For the detection of HER2 and HER3 by classic IHC, the Calu3 tumors are explanted form the animal and fixed in for 24 hours in formalin. Thereafter, the tumor tissue is dehydrated and embedded in paraffin. The tissue is cut, uptaken on glass slides, air dried over night at 37 °C followed by de-paraffinization and heat-induced epitope retrieval (Dako Target Retrieval Solution). For staining the HER2- and HER3-Receptor a anti-Her2 mouse mAb form Ventana (4B5) and anti-HER3 mouse mAb from Dako (anti-HER3) are used and it's performed as specified by the manufacturer. After that, the slides are covered with a cover glass and visualized by bright field microscopy. The percentage of positive cells and staining intensity are analyzed by an approved Pathologist. It resulted in a very high staining intensity of +++ and >95% of positive cells for HER2 and high staining intensity of ++-+++ and 90% positive cells for HER3. Thereby, IHC detection of the HER2- and HER3-Receptor's exhibit a high receptor expression level in Calu3 lung cancer xenograft (**Figure 3****.****1**).

For non-invasive detection of the HER2- and HER3-Receptor by near-infrared fluorescence imaging, three s.c. Calu3 tumor bearing mice are injected with a Cy5 labeled anti-HER2 mAb (Trastuzumab) and three are injected with Cy5 labeled anti-HER3 (Ab#6 described in WO2008100624(A2,A3)). After a single i.v. injection of 50 microgram of the labelled mAb, the fluorescence signal is imaged in the tumor area after 48 hours. The signals of all in vivo images are normalized, converted into pseudocolor and overlayed to the bright field image of the mice. The imaging results show for the anti-HER3-Cy5 mAb a very low and for the anti-HER2-Cy5 mAb a very strong signal intensity in the tumor region. It indicates that no or only a very low binding of anit-HER3-Cy5 mAb to the HER3-Receptor take place. In contrast, the anti-HER2-Cy5 mAb exhibit a strong affinity and binding to the HER2-Receptor (**Figure 3****.****2**).

The results from in vivo imaging are confirmed by histological ex vivo analysis. For this, all tumors are explanted and fixed in for 8 hours in formalin. Thereafter, the tumor tissue is dehydrated and embedded in paraffin. The tissue is cut, uptaken on glass slides, air dried in the dark over night at 37 °C. After de-paraffinization the slides are stained with DAPI and visualized by fluorescence microscopy (**Figure 3.3**). The results form ex vivo imaging correlate perfectly with the in vivo data. The low anti-HER3-Cy5 signal are only located in the murine tissue and no specific antibody binding to tumor cells are visible (**Figure 3.3** **a**)**.** On the other hand, a very strong and highly specific anti-HER2-Cy5 signal in visible around the Calu3 tumor cells (**Figure 3.3** **b**)**.**

The experimental proof of HER2 and HER3 in Calu3 by IHC and NIRF-Imaging lead to different results. IHC indicate a highly positive receptor expression for both receptors and consequently Calu3 should be a appropriate preclinical xenograft model for testing the antitumor efficacy of anti-HER2 and anti-HER3 mAb. In comparison the NIRF-Imaging of the therapeutic anti-HER3-Cy5 mAb show a weak and anti-HER2-Cy5 a very strong signal in the tumor area and would therefore priories Calu3 as a preclinical model for the therapeutic anti-HER2 mAb.

To confirm those previous data, anti-HER2 and anti-HER3 therapeutic antibodies are tested in a preclinical study. The s.c. Calu3 bearing SHO mice are divided in three groups with eight mice each. The untreated vehicle group (1) are injected i.p. with Histidin buffer once a week. The first treatment group (2) are injected i.p. with a therapeutic anti-HER2 antibody concentration of 10 mg/kg once a week. The second treatment group (3) are injected i.p. with a therapeutic anti-HER3 antibody concentration of 10 mg/kg once a week. The tumor volumes of all animals are measured over the time by calliper (**Figure 3****.****4**)**.**

The therapeutic anti-Her3 mAb possess no antitumor activity in Calu3 lung cancer xenografts and the therapeutic anti-Her2 mAb show a strong antitumor activity in Calu3 lung cancer xenografts. Both results confirm with NIRF-Imaging.

### Example 4

The next example compares the ability of Immunohistochemistry (IHC) and near-infrared fluorescence imaging (NIRF), to verify the expression level of the tumor associated HER2- and IGF1-Receptor's in Calu3 lung cancer xenograft.

The human lung adenocarcinoma cancer cell line Calu-3 was used in this study. Tumor cells (5.0 x 106 cells in 100 microliter PBS) were injected subcutaneously in BALB/c nude mice. At a tumor volume between 150 and 200 mm³ the Cy5 fluorescence labeled anti-HER2 (trastuzumab) and anti-IGF1R (cl18) monoclonal antibodies were injected i.v. (2 mg/kg) and in vivo imaging was generated with the imaging system MAESTRO from Cambridge Research Instruments. Immediately after the in vivo imaging tumor were explanted, fixed in formalin and embedded in paraffin. Slides were analyzed by multispectral fluorescence with the NUANCE fluorescence microscope (Cambridge Research Instruments)

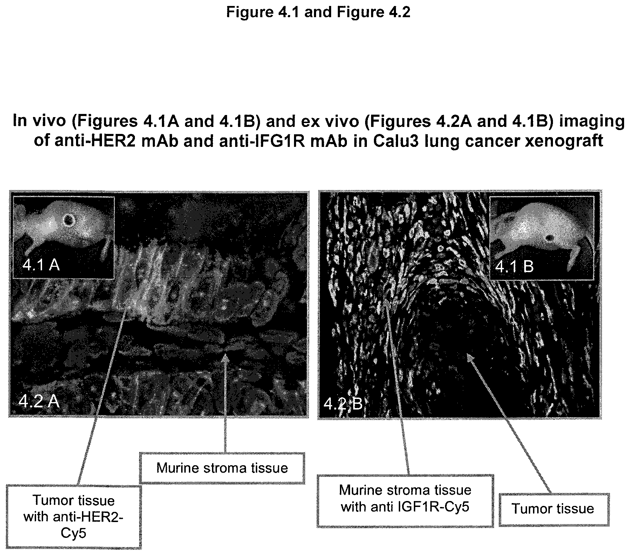

The in vivo imaging reveals comparable signals (generated from the fluorescence labeled antibodies) suggesting that both antibodies bind to tumor cells (Figure 4.1A and 4.1B). Specific binding of an antibody to tumor cells is a prerequisite for efficacy. Examination of explanted tumor tissue by multispectral fluorescence analysis reveals that the anti-HER2 monoclonal antibody binds specifically to the tumor cells (Figure 4.2A). Tumor cells were stained with the FISH detection kit according to the manufacturer's instructions. This test shows how many copies of the HER2 gene are in tumor cells. The more copies of the gene, the more HER2 receptors the cells have (green dots). However, the cl18 monoclonal antibody targeting the IGF1 receptor did not bind to tumor cells, but is located in the mouse stroma tissue (Figure 4.2B).

## Claims

1. A non-invasive method for identifying a cancer-patient disposed to respond favorably to a therapeutic antibody, which method comprises:
(a) directing excitation light of at least one predetermined wavelength onto a predetermined region of said patient,
(b) receiving emission light emitted from said predetermined region from a fluorescently labeled antibody with a wavelength distinguishable from to the predetermined wavelength of (a),
wherein said cancer-patient is one who had received said therapeutic antibody in a fluorescently labeled form prior to the identification;
and wherein the method further comprises detecting, ex vivo, the fluorescently labeled antibody and thereby a subset of tumor cells in a tissue sample obtained from said patient by direct or indirect immunohistochemistry, wherein said patient is one who had received the fluorescently labeled antibody prior to the removal of said tissue sample,
thereby identifying a cancer-patient whose tumor cells are labeled by said antibody, said patient being disposed to respond favorably to said therapeutic antibody.

2. The method of claim 1, wherein step (b) further comprises:
comparing the signal obtained from the cancer-patient with reference data, thereby determining a threshold level of fluorescence and identifying a cancer-patient whose tumor cells are labeled beyond said threshold level.

3. The method of claim 1 or 2, wherein said patient is one who has been diagnosed to suffer from cancer.

4. The method of any of claims 1 to 3, wherein said predetermined region of said patient comprises at least the breast, liver, a kidney, bladder, lung, prostate, or pancreas of said patient.

5. The method of any one of the preceding claims, wherein said cancer is breast-cancer.

6. The method of any one of the preceding claims, wherein said fluorescent label is selected from the group consisting of quantum dot agents, fluorescent dyes, pH-sensitive fluorescent dyes, voltage sensitive fluorescent dyes, and fluorescent labeled microspheres.

7. The method of any one of claims 1 to 6, wherein said subject has received said antibody in a therapeutically effective dose prior to the identification.

8. The method of claim 1, wherein said immunohistochemistry is **characterized by** the following steps:
(a) providing said tissue sample under conditions which are suitable for immunohistochemistry**;**
(b) optionally fixing said tissue sample;
(c) directly or indirectly detecting the antibody and thereby the subset of cells.

9. The method of claim 1, wherein the cancer-patient is a xenograft tumor model.

10. The method of 9, wherein said xenograft tumor model comprises a tumor.

11. The method of claim 10, wherein said tumor is from a human.

12. The method of any one of claims 9 to 11, wherein said xenograft tumor model is a mouse, rat, guinea pig, hamster or non-human monkey.

13. The method of any one of claims 9 to 12, further comprising the step of
i. detecting in said xenograft tumor model said therapeutic antibody after binding to its target in said xenograft tumor model by near-infrared fluorescence imaging (NIRF); and
ii. selecting the xenograft tumor model which shows after binding of said therapeutically effective antibody to said target in said xenograft tumor model the highest fluorescence signals.

## Patentansprüche

1. Nichtinvasives Verfahren zur Erkennung eines Krebspatienten, der disponiert ist, positiv auf einen therapeutischen Antikörper zu reagieren, wobei das Verfahren Folgendes umfasst:
(a) Richten von Anregungslicht von wenigstens einer vorgegebenen Wellenlänge auf einen vorgegebenen Bereich des Patienten,
(b) Empfangen von, von dem vorgegebenen Bereich emittiertem Emissionslicht von einem fluoreszierend markierten Antikörper mit einer Wellenlänge, die von der vorgegebenen Wellenlänge von (a) unterscheidbar ist,
wobei der Krebspatient ein Patient ist, der den therapeutischen Antikörper in fluoreszierend markierter Form vor der Erkennung erhalten hat;
und wobei das Verfahren weiterhin Folgendes umfasst: ex vivo Feststellung des fluoreszierend markierten Antikörpers und dadurch einer Untergruppe von Tumorzellen in einer Gewebeprobe, die man von dem Patienten durch direkte oder indirekte Immunhistochemie erhalten hat,
wobei der Patient ein Patient ist, der den fluoreszierend markierten Antikörper vor der Entnahme der Gewebeprobe erhalten hat,
wodurch ein Krebspatient erkannt wird, dessen Tumorzellen durch den Antikörper markiert sind, wobei der Patient disponiert ist, positiv auf den therapeutischen Antikörper zu reagieren.

2. Verfahren nach Anspruch 1, wobei Schritt (b) weiterhin Folgendes umfasst:
Vergleichen des von dem Krebspatienten erhaltenen Signals mit Referenzdaten, wodurch ein Fluoreszenz-Schwellenwert bestimmt wird und ein Krebspatient erkannt wird, dessen Tumorzellen über diesen Schwellenwert hinaus markiert sind.

3. Verfahren nach Anspruch 1 oder 2, wobei der Patient ein Patient ist, bei dem Krebs diagnostiziert wurde.

4. Verfahren nach einem der Ansprüche 1 bis 3, wobei der vorgegebene Bereich des Patienten wenigstens die Brust, Leber, eine Niere, die Blase, die Lunge, die Prostata oder die Bauchspeicheldrüse des Patienten umfasst.

5. Verfahren nach einem der vorhergehenden Ansprüche, wobei der Krebs Brustkrebs ist.

6. Verfahren nach einem der vorhergehenden Ansprüche, wobei die Fluoreszenzmarkierung ausgewählt ist aus der Gruppe, bestehend aus Quantenpunkt-Wirkstoffen, Fluoreszenzfarbstoffen, pH-empfindlichen Fluoreszenzfarbstoffen, spannungsempfindlichen Fluoreszenzfarbstoffen und fluoreszierend markierten Mikrosphären.

7. Verfahren nach einem der Ansprüche 1 bis 6, wobei der Patient vor der Erkennung einen Antikörper in wirksamer Dosis erhalten hat.

8. Verfahren nach Anspruch 1, wobei die Immunhistochemie durch die folgenden Schritte gekennzeichnet ist:
(a) Bereitstellen der Gewebeprobe unter Bedingungen, die für die Immunhistochemie geeignet sind;
(b) wahlweise Fixieren der Gewebeprobe;
(c) direkte oder indirekte Erkennung des Antikörpers und dadurch der Untergruppe von Zellen.

9. Verfahren nach Anspruch 1, wobei der Krebspatient ein Xenotransplantat-Tumormodell ist.

10. Verfahren nach Anspruch 9, wobei das Xenotransplantat-Tumormodell einen Tumor umfasst.

11. Verfahren nach Anspruch 10, wobei der Tumor von einem Menschen stammt.

12. Verfahren nach einem der Ansprüche 9 bis 11, wobei das Xenotransplantat-Tumormodell eine Maus, eine Ratte, ein Meerschweinchen, ein Hamster oder ein nichtmenschlicher Affe ist.

13. Verfahren nach einem der Ansprüche 9 bis 12, weiterhin umfassend die folgenden Schritte:
i. Erkennen des therapeutischen Antikörpers in dem Xenotransplantat-Tumormodell nach Binden an sein Target in dem Xenotransplantat-Tumormodell durch Nahinfrarot-Fluoreszenzabbildung (NIRF); und
ii. Auswählen des Xenotransplantat-Tumormodells, das nach dem Binden des therapeutisch wirksamen Antikörpers an das Target in dem Xenotransplantat-Tumormodell die höchsten Fluoreszenzsignale zeigt.

## Revendications

1. Procédé non invasif pour identifier un patient atteint d'un cancer susceptible de répondre favorablement à un anticorps thérapeutique, le procédé comportant:
(a) diriger de la lumière d'excitation d'au moins une longueur d'onde prédéterminée sur une région prédéterminée dudit patient,
(b) recevoir de la lumière d'émission émise par ladite région prédéterminée d'un anticorps marqué par fluorescence avec une longueur d'onde distinguable de la longueur d'onde prédéterminée de (a),
ledit patient atteint d'un cancer étant quelqu'un qui a reçu ledit anticorps thérapeutique sous une forme marqué de façon fluorescente avant l'identification;
et le procédé comportant en outre la détection *ex vivo* de l'anticorps marqué par fluorescence et ainsi d'un sous-ensemble des cellules tumorales dans un échantillon de tissu obtenu dudit patient par immunohistochimie directe ou indirecte, ledit patient étant quelqu'un qui avait reçu l'anticorps marqué par fluorescence avant le prélèvement dudit échantillon de tissu,
ainsi identifiant un patient atteint d'un cancer dont les cellules tumorales sont marquées par ledit anticorps, ledit patient étant susceptible de répondre favorablement audit anticorps thérapeutique.

2. Procédé selon la revendication 1, l'étape (b) comportant en outre:
la comparaison du signal obtenu du patient atteint d'un cancer avec des données de référence, ainsi déterminant un niveau seuil de fluorescence et identifiant un patient atteint d'un cancer dont les cellules tumorales sont marquées au-delà dudit niveau seuil.

3. Procédé selon la revendication 1 ou 2, ledit patient étant quelqu'un diagnostiqué comme étant atteint du cancer.

4. Procédé selon l'une quelconque des revendications 1 à 3, ladite région prédéterminée dudit patient comportant au moins le sein, le foie, un rein, la vessie, le poumon, la prostate ou le pancréas dudit patient.

5. Procédé selon l'une quelconque des revendications précédentes, ledit cancer étant le cancer du sein.

6. Procédé selon l'une quelconque des revendications précédentes, ledit marqueur fluorescent étant choisi parmi le groupe constitué des agents de *quantum dots,* des colorants fluorescents, des colorants fluorescents sensibles au pH, des colorants fluorescents sensibles à la tension, et des microsphères marquées par fluorescence.

7. Procédé selon l'une quelconque des revendications 1 à 6, ledit sujet ayant reçu ledit anticorps dans une dose thérapeutiquement efficace avant l'identification.

8. Procédé selon la revendication 1, ladite immunohistochimie étant **caractérisée par** les étapes suivantes:
(a) fournir ledit échantillon de tissu dans des conditions appropriées pour l'immunhistochimie;
(b) facultativement fixer ledit échantillon de tissu;
(c) détecter directement ou indirectement l'anticorps et ainsi le sous-ensemble des cellules.

9. Procédé selon la revendication 1, le patient atteint d'un cancer étant un modèle de tumeur xénogreffe.

10. Procédé selon la revendication 9, ledit modèle de tumeur xénogreffe comportant une tumeur.

11. Procédé selon la revendication 10, ladite tumeur étant une tumeur humaine.

12. Procédé selon l'une quelconque des revendications 9 à 11, ledit modèle de tumeur xénogreffe étant une souris, un rat, un cobaye, un hamster ou un singe non humain.

13. Procédé selon l'une quelconque des revendications 9 à 12, comportant en outre les étapes suivantes:
i. détecter dans ledit modèle de tumeur xénogreffe ledit anticorps après liaison à sa cible dans ledit modèle de tumeur xénogreffe par l'imagerie de fluorescence dans l'infrarouge proche (NIRF); et
ii. choisir le modèle de tumeur xénogreffe qui montre les signaux de fluorescence les plus élevés après liaison dudit anticorps thérapeutiquement efficace à ladite cible dans ledit modèle de tumeur xénogreffe.
